# EUROPEAN PATENT SPECIFICATION

(11) **EP 2 908 850 B1**
(45) Date of publication and mention of the grant of the patent: **07.03.2018**
(21) Application number: 13791744.9
(22) Date of filing: 17.10.2013
(51) Int. Cl.: A61K 38/47, A61K 31/7028, A61P 25/00, A61P 25/28

(54) **A COMPOSITION COMPRISING GLUCORAPHANIN, MYROSINASE AND A BUFFERED SOLUTION FOR USE IN THE TREATMENT OF NEURODEGENERATIVE DISEASES**
EINE ZUSAMMENSETZUNG ENTHALTEND GLUCORAPHANIN, MYROSINASE UND EINE PUFFERLÖSUNG ZUR BEHANDLUNG VON NEURODEGENERATIVEN ERKRANKUNGEN
UNE COMPOSITION COMPRENANT DE LA GLUCORAPHANINE, LA MYROSINASE ET UNE SOLUTION TAMPON POUR LE TRAITEMENT DES MALADIES NEURODÉGÉRATIVES

(30) Priority: 19.10.2012 IT MI20121774
(43) Date of publication of application: 26.08.2015
(73) Proprietor: Bramanti, Placido, 98123 Messina (IT); Mazzon, Emanuela, 33080 Prata di Pordenone (IT); Iori, Renato, 41013 Castelfranco Emilia (IT)
(72) Inventor: DE NICOLA, Gina Rosalinda, I-51100 Pistoia (IT)
(74) Representative: Gervasi, Gemma
(86) International application number: PCT/EP2013/071706
(87) International publication number: WO 2014/060509

(56) References cited:
- EP-A1- 2 213 280
- WO-A1-2009/083871
- WO-A1-2012/074412
- WO-A2-03/051313
- WO-A2-2012/037193
- US-A1- 2002 091 087
- US-A1- 2012 213 890
- US-A1- 2012 258 922
- Morroni et al.: "Sulforaphane improves impairments and protects against neuron death in a mouse model of Parkinson's disease", 35 Congresso Nazionale della Societa Italiana di Farmacologia , 14 September 2011 (2011-09-14), XP002690918, Retrieved from the Internet: URL:http://cong35.sifweb.org/congresso_abs _view.php?id=542 [retrieved on 2013-01-22]
- KELSEY NATALIE A ET AL: "Nutraceutical antioxidants as novel neuroprotective agents.", MOLECULES (BASEL, SWITZERLAND) NOV 2010, vol. 15, no. 11, November 2010 (2010-11), pages 7792-7814, XP002690919, ISSN: 1420-3049
- PING Z ET AL: "Sulforaphane protects brains against hypoxic-ischemic injury through induction of Nrf2-dependent phase 2 enzyme", BRAIN RESEARCH, ELSEVIER, AMSTERDAM, NL, vol. 1343, 9 July 2010 (2010-07-09), pages 178-185, XP027096579, ISSN: 0006-8993 [retrieved on 2010-04-24]
- HAN JI MAN ET AL: "Protective effect of sulforaphane against dopaminergic cell death", JOURNAL OF PHARMACOLOGY AND EXPERIMENTAL THERAPEUTICS, AMERICAN SOCIETY FOR PHARMACOLOGY AND EXPERIMENTAL THERAPEUTICS, US, vol. 321, no. 1, April 2007 (2007-04), pages 249-256, XP002518062, ISSN: 0022-3565, DOI: 10.1124/JPET.106.110866
- ZHAO J ET AL: "Sulforaphane reduces infarct volume following focal cerebral ischemia in rodents", NEUROSCIENCE LETTERS, LIMERICK, IE, vol. 393, no. 2-3, 30 January 2006 (2006-01-30), pages 108-112, XP027885485, ISSN: 0304-3940 [retrieved on 2006-01-30]

## Description

### FIELD OF THE INVENTION

The present invention concerns the use of a bioactivated phytochemical as a neuroprotective agent for preventing and treating neurodegenerative diseases, such as alterations in the permeability of the blood brain barrier tight junction, vascular dementia, brain ischemia/reperfusion injury, Parkinson disease, Alzheimer disease, multiple sclerosis, amyotrophic lateral sclerosis and Huntington disease. In particular, said phytochemical is *R_{S}*-Glucoraphanin bioactivated with myrosinase and has proved to be particularly suitable as being surprisingly effective and extremely well tolerated *in vivo.*

### STATE OF THE ART

Neurodegeneration is the umbrella term for the progressive loss of structure or function of neurons, including death of neurons. Many neurodegenerative diseases, including Parkinson disease, Alzheimer disease, multiple sclerosis, Amyotrophic Lateral Sclerosis and Huntington disease, occur as a result of neurodegenerative processes. As research progresses, many similarities appear which relate these diseases to one another on a sub-cellular level. Discovering these similarities offers hope for therapeutic advances that could ameliorate many diseases simultaneously.

Particularly, Parkinson's disease (PD) is a motor system disorder that affects between 100 and 200 per 100,000 people over 40, more commonly men than women. The incidence of the disease increases rapidly over 60 years, with a mean age at diagnosis of 70.5 years.

As neurodegenerative disorder, PD is characterized by progressive and selective loss of dopaminergic (DA) neurons in the substantia nigra pars compacta (SNpc) and striatum, accompanied by the appearance of Lewy bodies, abnormal aggregates mainly composed by alpha-synuclein, a mutated protein found in this as well as many other neurodegenerative diseases.

Clinically, resting tremor, rigidity, bradykinesia, and postural instability (difficulty with walking and gait) later followed by dementia, cognitive and behavioral problems are all symptoms that occur in the course of the PD.

The mechanisms regulating neuronal cell death in PD have been extensively investigated. Oxidative stress, protein misfolding and aggregation are involved in the pathogenesis of the disease.

In particular, a deficit in dopamine seems correlated with physiopathology of the disease. 1-Methyl-4-phenyl-1,2,3,6-tetrahydropyridine (MPTP)-induced neurotoxicity is the most common experimental model used through which mice shows typical neuropathological defects as observed in human PD. MPTP is converted to 1-methyl-4-phenylpyridinium (MPP+) by monoamine oxidase B and it is carried by the dopamine transporter (DAT) into DA cells, causing the block of mitochondrial complex I activity.

Neurodegenerative potential of MPTP-administration depends by the dosage. In particular, the administration of the dose of 40mg/kg (repeated twice) generates a model of acute PD, while 5 doses of 20mg/kg administration of MPTP reproduce a model of sub-acute PD.

Multiple sclerosis (MS) is a progressive inflammatory and demyelinating disease of the central nervous system (CNS) caused by malfunction of the immune system, that affects more than 2.5 million people worldwide every year, typically in adults between 20 to 45 years of age; affecting more women than men and Northern European people shown to be at highest risk for MS. MS attacks the myelinated axons in the CNS, destroying the myelin and the axons to varying degrees. Traditional literature has been thought that MS is an inflammatory disease primarily localized to the white matter of the brain and spinal cord. The clinical signs of MS are highly variable. MS patients often have symptoms of upper motor neuron disease that include hyperreflexia, ataxia, spasticity, and visual defects. In some cases there is evidence of lower motor neuron disease such as sensory defects and partial or complete paralysis.

The etiology of MS is yet unknown, but it appears to involve a combination of genetic susceptibility and a nongenetic trigger, such as a virus, metabolism, or environmental factors, that together result in a self-sustaining autoimmune disorder that leads to recurrent immune attacks against CNS. To better understand the etiopathogenesis of MS, researchers use some experimental models and, among these, one of the most used is the experimental autoimmune encephalomyelitis (EAE). EAE is a well characterized animal model, showing many clinical and pathological features of human MS, such as paralysis, weight loss, demyelination, and CNS inflammation.

Convincing evidences, supported by both animal and cell line studies, have demonstrated that regular consumption of *Brassicaceae* vegetables (broccoli, cabbage, cauliflower and Brussels sprouts, etc.) can contribute to reduced risk of neurodegenerative disorders and cancer. It is believed that the benefit of these vegetables are due to their high content of specific phytochemicals, known as glucosinolates (GLs).

Their structures share a common core of a β-D-glucopyrano moiety linked via a sulfur atom to a (Z)-N-hydroximinosulfate ester and a variable aglycon side chain derived from the α-amino acid biosynthetic precursor. Several methionine-derived GLs, which constitute the largest group of GLs, bear in their side chain an additional sulfur atom at different oxidation states (sulfide, sulfoxide, or sulfone functions).

GLs coexist in the same plant, but in separate cells, with the enzyme β-thioglucoside glucohydrolase, usually known as myrosinase (Myr; EC 3.2.1.147), which have also been found within human bowel microflora. After mechanical damage of cells, e.g. predation/mastication by humans or animals, freeze-thaw injury or plant pathogens, GLs are hydrolyzed releasing, apart from glucose and sulfate, several biologically active compounds such as isothiocyanates (ITCs), thiocyanates and nitriles, depending on the hydrolytic conditions. At neutral pH condition Myr catalyses the GLs hydrolysis (>99%) producing the corresponding ITCs.

To date, most studies on the biological activities of the ITC sulforaphane (SFN) have been conducted by using the racemic form of the synthetic sulforaphane (*R_{S}*,*S_{S}*-SFN). *R_{S}*,*S_{S}*-SFN has been extensively studied in recent years, both as chemopreventive agent and as potential novel chemotherapeutic compound, but this compound currently used is unstable and slightly soluble in water, thus the object of the current invention is to provide a therapeutic agent of easier availability, and at the same time, very effective in preventing and treating neurodegenerative diseases.

### SUMMARY

This object was achieved by the composition comprising *R_{S}*-Glucoraphanin bioactivated with myrosinase for use as a neuroprotective agent for preventing and treating neurodegenerative diseases, i.e. diseases in which the nervous system progressively and irreversibly deteriorates. For the purposes of the present disclosure with the phrase "composition comprising *R_{S}*-Glucoraphanin bioactivated with myrosinase" is meant a composition comprising *R_{S}*-Glucoraphanin and myrosinase, and is also hereinafter shortly referred to as "bioactive *R_{S}*-GRA".

In another aspect, the present disclosure concerns a kit for providing said composition, the kit comprising:
a) a container comprising *R_{S}*-Glucoraphanin,
b) a container comprising myrosinase, and
c) a leaflet comprising instructions for simultaneous, separate or sequential use of *R_{S}*-Glucoraphanin and myrosinase, in preventing and treating neurodegenerative diseases.
The invention is defined in the claims.

### BRIEF DESCRIPTION OF THE DRAWINGS

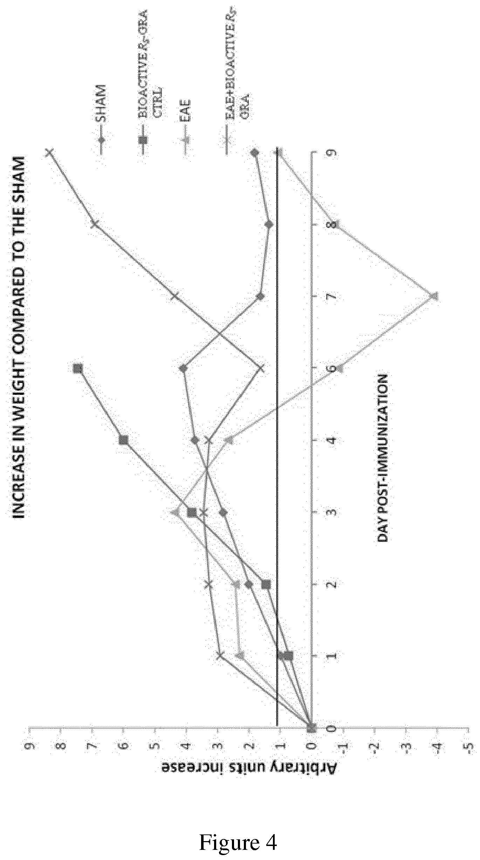

The characteristics and advantages of the present invention will be evident from the detailed description given below, as well as the working examples provided for illustrative and nonlimiting purposes, and the annexed Figures, wherein:
- *Figure 1*: chemical structure and enzymatic reaction for bioactivation of *R_{S}*-GRA by Myr. (*R_{S}*)-glucoraphanin purified from Tuscan black kale seed, bio-activated by myrosinase enzyme: *In situ* release of (*R_{S}*)-sulforaphane.
- *Figure* 2: bioactive *R_{S}*-GRA-treatment prevents body weight loss and preserves DA neurons by MPTP-induced damage. See Example 1
   Body weight (A) and behavioral test is performed to assess bioactive *R_{S}*-GRA-neuroprotection in sub-acute and acute MPTP-administrated mice.
   Sub-acute MPTP-administrated mice group shows a higher body weight at the beginning of observation time, (day 1) (A), but, causing the progression of the disease, the body weight of 20 mg/kg MPTP-administrated mice rapidly and continuously decreases up to the end of the experimental period. In particular, after the fourth dose of MPTP, there is a consistent difference among PD group and all other groups (A). On the contrary, after PD induction, bioactive *R_{S}*-GRA-treated mice increase their body weight (A).
   No sign of any decrease in body weight is detected in bioactive *R_{S}*-GRA-treated control group (A).
   Open field test is performed separately and in a different way for 40mg/kg (without latency-time) (B) and 20mg/kg (with latency-time) (C, C1) MPTP set groups.
   In acute MPTP-administrated animals, a reduced degree of mobility and a little number of changes is noted (B), while bioactive *R_{S}*-GRA-treatment after MPTP-administration preserve the mice by the neurological changes found in the behavior (B) also compared to the results of naive and bioactive *R_{S}*-GRA-control group (B).
   During latency-time, 20mg/kg MPTP-administrated mice causes an immobility of around 40sec (C), while the latency-time of bioactive *R_{S}*-GRA-treated is around equal to zero (C). Conversely, confirming this data, during open field 20mg/kg MPTP-administrated mice totalize a total number of changed fields lower compared to the bioactive *R_{S}*-GRA-treated group (C1). The same experimental group examined in a pole test gives significant results. 20mg/kg MPTP-administrated mice treated with bioactive *R_{S}*-GRA show a reduced bewilderment when placed head-up on top of the vertical wooden pole and compared with the untreated groups (D). Moreover, mice pharmacologically injected with bioactive *R_{S}*-GRA show a faster drop time, if the results are compared with sub-acute MPTP-administrated group that doesn't receive this treatment.
   Statistical analysis legend:
   OPEN FIELD 40mg/kg (B) # p<0.05 vs MPTP 40mg/kg; ***p<0.0006 vs NAÏVE;
   LATENCY (C) *p<0.05 vs MPTP 20mg/kg;
   OPEN FIELD 20mg/kg (C1) **p<0.005 vs MPTP 20mg/kg
   POLE TEST MPTP 20mg/kg (D) *p<0.05 vs bioactive *R_{S}*-GRA-control group;

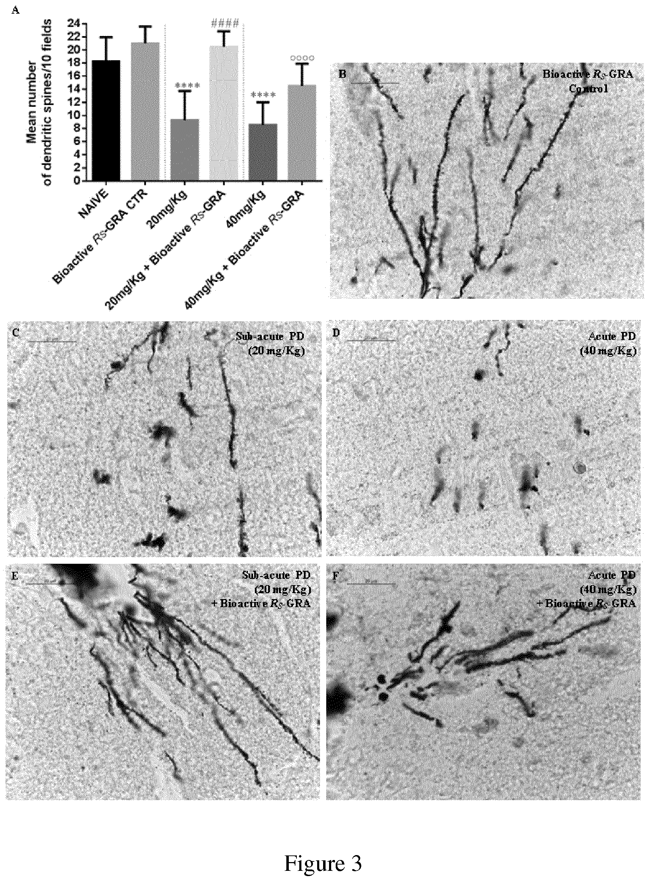

- *Figure 3**:* Golgi stain for dendritic spine detection shows a marked recovery in bioactive *R_{S}*-GRA-treated mice in PD mice. See Example 1
   Brain samples taken from bioactive *R_{S}*-GRA-control animals show a normal number of dendritic spines (B). Degree of MPTP lesions in the SNpc is scored on the basis of neuronal dendritic spine disruption. Nigral DA neurons are preserved by bioactive *R_{S}*-GRA-treatment in sub-acute (E) and (F) MPTP-injured mice sections. A severe loss of termination at level of the dendritic tree is marked in sub-acute (C) MPTP-damaged section and more significantly evident in acute (D) MPTP-administration when bioactive *R_{S}*-GRA wasn't administrated.
   Bioactive *R_{S}*-GRA-treatment (A) preserves dendritic spines by neuronal degeneration following sub-acute and acute MPTP-administration.
   Statistical analysis legend:
   DENDRITIC SPINES (A) *p<0.05 vs NAÏVE; **p<0.05 vs NAÏVE; ****p<0.0001 vs NAÏVE; ####p<0.0001 vs 20mg/kg MPTP; °°°°p<0.0001 vs 40mg/kg MPTP
- *Figure 4**:* Body weight gain/loss. See Example 2
   C57BL/6 mice were immunized with MOG₃₃₋₅₅ and then treated daily with bioactive *R_{S}*-GRA at the dose of 10 mg/kg. Mice were monitored daily for weight gain/loss. After EAE induction, significative body weight loss was observed in EAE mice. Also a significative body weight gain was found in EAE+ bioactive *R_{S}*-GRA group and in bioactive *R_{S}*-GRA-control group mice compared with EAE group and sham group.

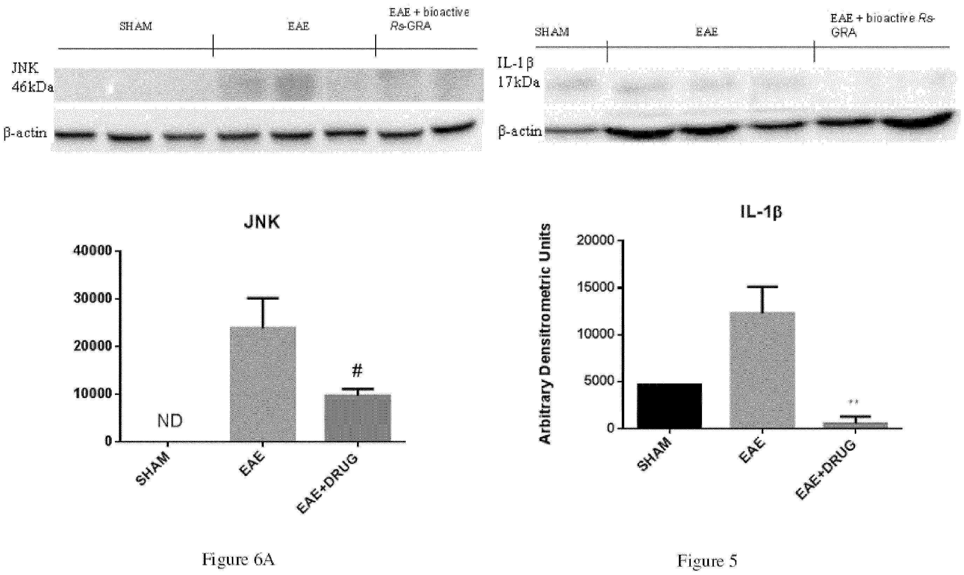

- *Figure 5**:* Western Blot for IL-1β. See Example 2
   By Western Blot analysis, a basal level of IL-1β was detected in spinal cord samples collected from EAE mice 7 days after EAE, while spinal cord levels of IL-1β were attenuated by administration of bioactive *R_{S}*-GRA. β-actin was used as internal control. **P <0,0072 vs. EAE.

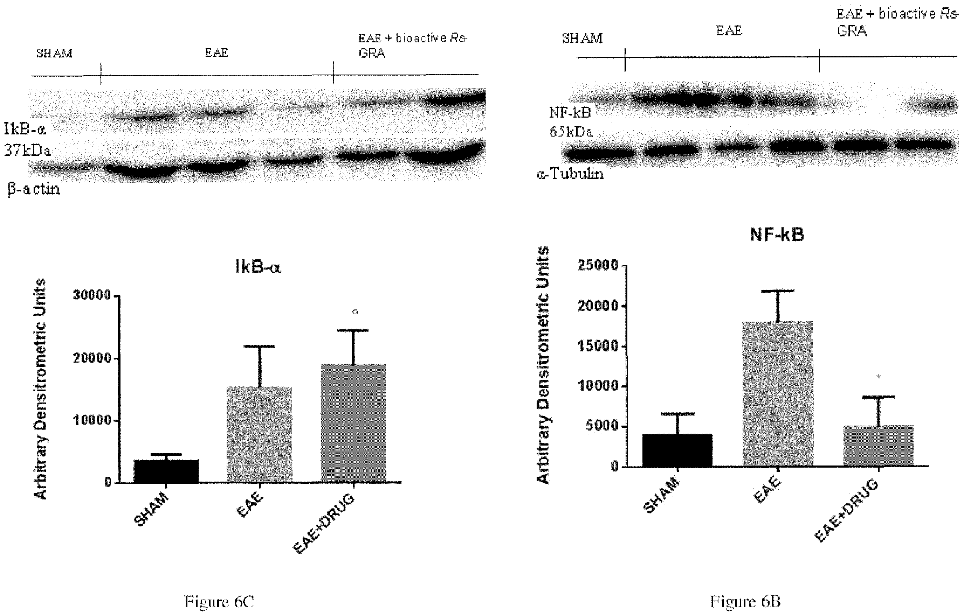

- *Figure* 6: Western Blot for JNK, NF-kB and IkB-a. See Example 2
   By Western blot analysis, basal expression of JNK was detected in the spinal cord samples from sham animals, whereas JNK levels were substantially increased in EAE mice (A). Bioactive *R_{S}*-GRA treatment prevented the EAE-induced JNK expression (A). In addition, EAE caused a significant increase in nuclear NF-kB p65 compared to the sham mice (B). Bioactive *R_{S}*-GRA treatment reduced the levels of NF-kB p65 (B). Also, a basal level of IkB-a was detected in the spinal cord samples from EAE animals and in sham mice (C). Bioactive *Rs*-GRA administration prevented the EAE-induced IkB-a degradation (C). The relative expression of the protein bands was standardized for densitometric analysis to β-actin and α-tubulin levels. *P< 0.008 vs. EAE, ^{#}P<0.0021 vs. EAE, °P<0.0374 vs. EAE.

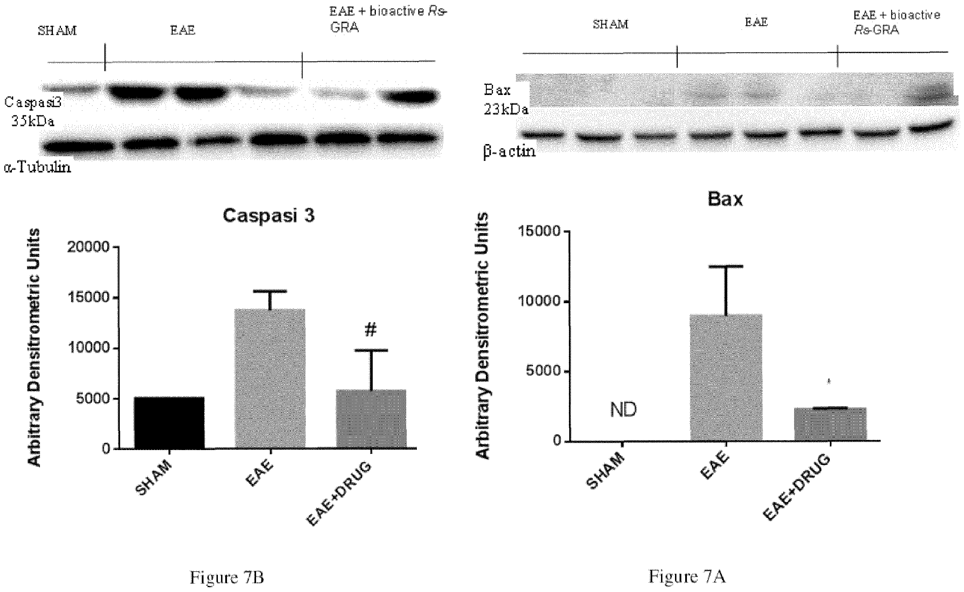

- *Figure 7*: Western Blot for Bax and Caspase 3. See Example 2
   Representative Western blots showing no significant Bax expression in spinal cord tissues from sham mice (A). Bax levels were appreciably increased in spinal cord from EAE mice (A). Also, spinal cord levels of Bax were attenuated by administration of bioactive *R_{S}*-GRA (A). Moreover, it was also demonstrated Caspase 3 activation by Western blot, EAE caused a significant increase in Caspase 3 expression (B) compared to the sham mice. Bioactive *R_{S}*-GRA reduced Caspase 3 levels as shown in figure (B). β-actin and α-tubulin were used as internal control.*P<0.0110 vs. EAE, ^{#}P<0.0269 vs. EAE.

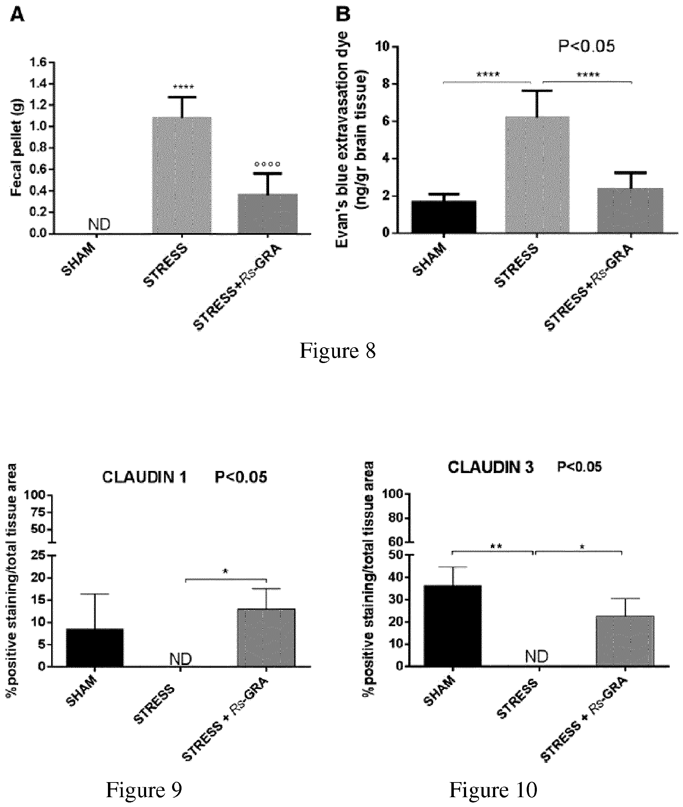

- *Figure* 8: Effects of RS-GRA on stress parameters. See Example 3
   (A) Fecal pellet output collected during the immobilization period was significantly higher than sham group (****P value < 0.0001). Moreover, the treatment with bioactive *R_{S}*-GRA considerably decreased fecal pellet production in stressed mice (vs. non-treated stress group, °°°°P value < 0.0001). The results were obtained by one-way ANOVA test followed by a Bonferroni post hoc test. (B) Evan's blue dye following the induction of restraint stress shows that stressed mice have severe levels of extravasation, indicating a BBB modified permeation, while mice administered with bioactive *R_{S}*-GRA show a significant decrease in Evan's blue penetration. Sham animals show a very low detection of Evan's blue dye.
- *Figure 9**:* Effect of bioactive *R_{S}*-GRA treatment on claudin-1 distribution. Densitometric analysis is shown: a P value < 0.05 was considered statistically significant. See Example 3
- *Figure 10**:* Effect of bioactive *R_{S}*-GRA treatment on claudin-3 distribution. Densitometric analysis is shown: a P value < 0.05 was considered statistically significant. See Example 3

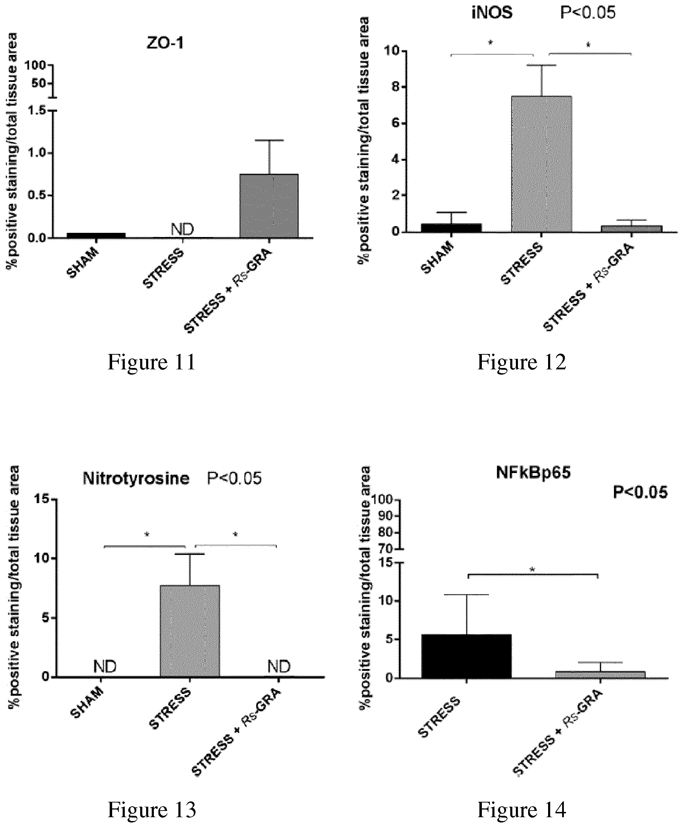

- *Figure 11**:* Effect of bioactive *R_{S}*-GRA treatment on ZO-1 distribution. Densitometric analysis is shown: a P value < 0.05 was considered statistically significant. See Example 3
- *Figure 12**:* Bioactive *R_{S}*-GRA modulates expression of iNOS. Densitometric analysis is provided: a P value < 0.05 was considered statistically significant. See Example 3
- *Figure 13**:* Effect of bioactive *R_{S}*-GRA on nitrotyrosine formation. Densitometric analysis is provided: a P value < 0.05 was considered statistically significant. See Example 3
- *Figure 14**:* Effect of bioactive *R_{S}*-GRA treatment on NF-kB localization. Densitometric analysis is shown: a P value < 0.05 was considered statistically significant. See Example 3

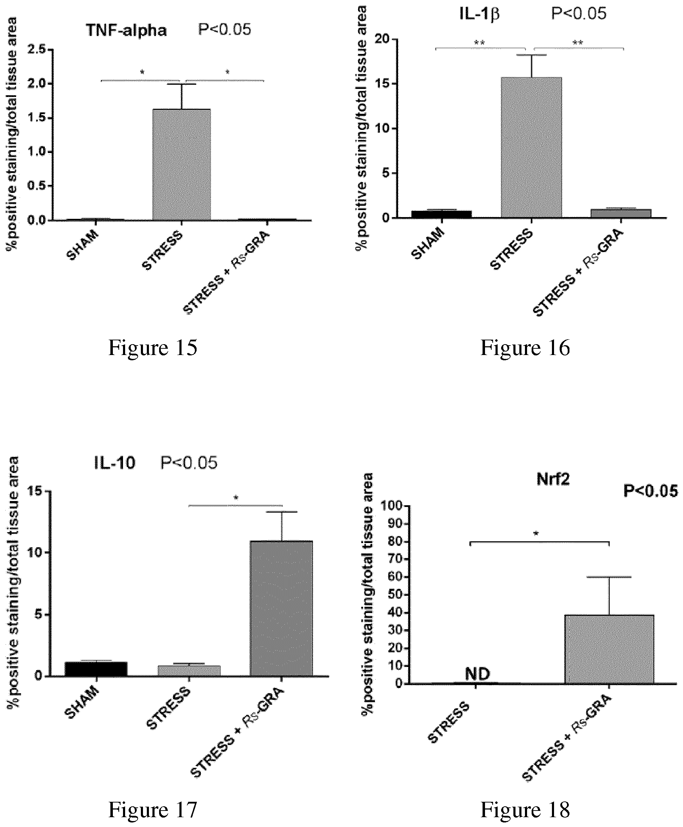

- *Figure 15**:* Effect of bioactive *R_{S}*-GRA treatment on TNF-α expression. Densitometric analysis is shown: a P value < 0.05 was considered statistically significant. See Example 3
- *Figure 16**:* Effect of bioactive *R_{S}*-GRA treatment on IL-1β expression. Densitometric analysis is shown: a P value < 0.05 was considered statistically significant. See Example 3
- *Figure 17*: Effect of bioactive *R_{S}*-GRA treatment on IL-10 expression. Densitometric analysis is shown: a P value < 0.05 was considered statistically significant. See Example 3
- *Figure 18*: Effect of bioactive *R_{S}*-GRA treatment on the Nrf2 localization. Densitometric analysis is shown: a P value < 0.05 was considered statistically significant. See Example 3

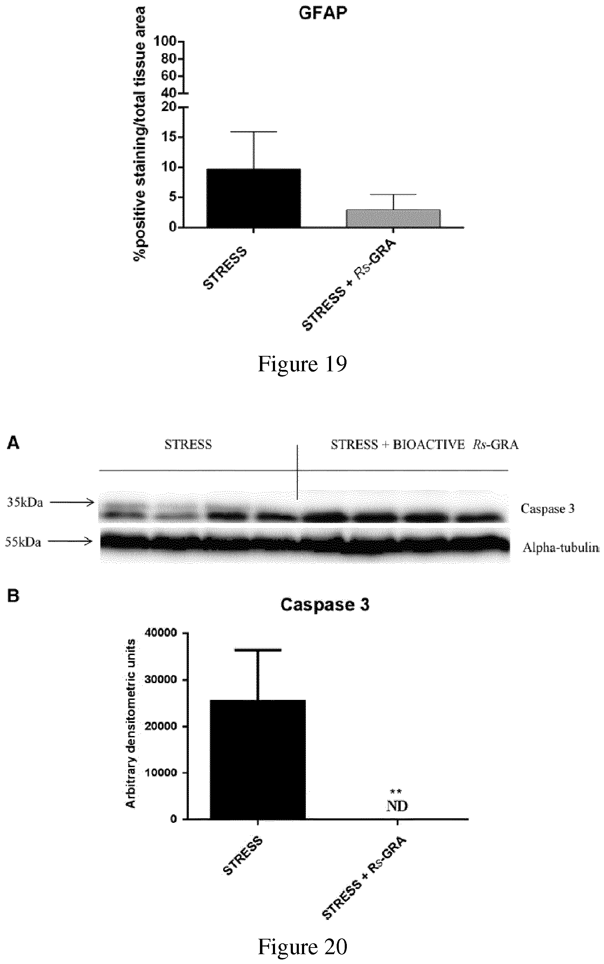

- *Figure 19**:* Effect of bioactive *R_{S}*-GRA treatment on GFAP expression. Densitometric analysis is shown: a P value < 0.05 was considered statistically significant. See Example 3
- *Figure 20**:* Effects of bioactive *R_{S}*-GRA treatment on caspase 3 expression. Representative western blotting of caspase 3 levels was realized in brain tissues. (A) At 150 min after immobilization, a significant increase in caspase 3 expression was observed in the sample obtained from stressed mice (A) compared with the bioactive *R_{S}*-GRA-treated mice (A); arbitrary densitometric analysis for caspase 3 was assessed (B). Data are expressed as arbitrary densitometric units (**P value < 0.05). The results were obtained by performing unpaired Student's t-test. See Example 3

### DETAILED DESCRIPTION OF THE INVENTION

The present invention thus concerns a composition comprising *R_{S}*-Glucoraphanin and myrosinase (Myr) for use as a neuroprotective agent for preventing and treating neurodegenerative diseases.

Particularly, said neurodegenerative diseases involve alterations in the permeability of the blood brain barrier tight junction, vascular dementia, brain ischemia/reperfusion injury, Parkinson's disease (PD) and PD-related disorders, Alzheimer's disease (AD) and other dementias, Prion disease, Motor neurone diseases (MND), Spinocerebellar ataxia (SCA), Spinal muscular atrophy (SMA), multiple sclerosis, amyotrophic lateral sclerosis and Huntington disease (HD). Reperfusion injury is the tissue damage caused when blood supply returns to the tissue after a period of ischemia or lack of oxygen. The absence of oxygen and nutrients from blood during the ischemic period creates a condition in which the restoration of circulation results in inflammation and oxidative damage through the induction of oxidative stress rather than restoration of normal function. The expression "brain ischemia/reperfusion injury" is used to shortly refer to this damage.

Preferably, said diseases are alterations in the permeability of the blood brain barrier tight junction, vascular dementia, brain ischemia/reperfusion injury, Parkinson disease, Alzheimer disease, multiple sclerosis, amyotrophic lateral sclerosis and Huntington disease.

As it will be evident from the Examples below, the composition of the invention comprising *R_{S}*-Glucoraphanin bioactivated with myrosinase (or shortly 'bioactive *R_{S}*-GRA'), has proved to be surprisingly and advantageously suitable as a neuroprotective agent, since it shows very effective antioxidant and anti-inflammatory properties.

In particular, it was shown a beneficial role of this bioactivated phytochemical during experimental brain ischemia/reperfusion injury, where bioactive *R_{S}*-GRA was given 15 min after ischemia (maximum peak derived by oxidative damage).

The neuroprotective role of the bioactive *R_{S}*-GRA was proved in an experimental rat model of brain ischemia/reperfusion injury (I/R). The mechanism underlying the inhibitory effects of bioactive *R_{S}*-GRA on inflammatory and apoptotic responses, induced by carotid artery occlusion in rats, was carefully examined. Cerebral I/R was induced by clamping of carotid artery for 1h, followed by 40 min of reperfusion through the release of the clamp. The achieved results clearly have shown that administration of bioactive *R_{S}*-GRA, 15 min after ischemia, significantly reduces proinflammatory parameters, such as inducible nitric oxide synthase expression (iNOS), adhesion molecules (ICAM-1), nuclear factor (NF)-kB traslocation as well as the triggering of the apoptotic pathway (TUNEL and Caspase 3 expression).

Taken together these data have shown that bioactive *R_{S}*-GRA possesses beneficial neuroprotective effects in the counteracting the brain damage associated to I/R. Therefore, bioactive *R_{S}*-GRA, could be an useful treatment in the cerebral ischemic stroke.

Moreover, in a model of restraint stress by immobility, well known to generate alterations of junction complexes of epithelia and endothelia, such as tight junction, bioactive *R_{S}*-GRA, preventively administered, protects the Blood Brain Barrier (BBB) from the alteration of the perm-selectivity. In fact, the bioactive *R_{S}*-GRA was tested in a mouse model of restraint stress in order to evaluate the capacity to prevent the dysfunction of BBB, structure fundamental for brain homeostasis. It is known that changes in BBB permeability were due to the disorganization of the Tight Junctions (TJs), large multiprotein complexes important for the maintenance of structural integrity and for the perm-selectivity of the barrier. Inflammation, oxidative stress, toxin, drugs and hormones have been shown to affect the TJ barrier and fence functions. In oxidative stress conditions, reactive oxygen species (ROS) overproduction causes TJ modulation and structural changes. Moreover, elevated glucocorticoid and glutamate levels trigger oxidative stress through the production of ROS, resulting into lipid peroxidation, protein oxidation, DNA damage, and cell death. By western blotting and immunohistochemical analysis it was demonstrated that immobilization stress leads to the alteration of TJs components, such as Claudin-1, Claudin-3 and ZO-1. Moreover, it was shown that these alterations were accompanied by an enhancement of Glial Fibrillary Acidic Protein (GFAP), an increase of NF-kB p65 activation and IkB-alpha degradation, as well as an overexpression of caspase 3. The achieved results revealed that bioactive *R_{S}*-GRA treatment significantly preserves the TJ integrity through an anti-oxidant effect.

Furthermore, the neuroprotective and immunomodulatory effects of bioactive *R_{S}*-GRA was proved in an Experimental Autoimmune Encephalomyelitis (EAE), a model of Multiple Sclerosis (MS), induced by immunization with myelin oligodendroglial glycoprotein peptide (MOG33-55) in mice.

By Western Blot analysis of spinal cord tissues, it was demonstrated that treatment with bioactive *R_{S}*-GRA significantly decreased nuclear factor (NF)-kB traslocation, pro-inflammatory cytokines production such as interleuchin-1β (IL-1β), and apoptosis (Bax and Caspase 3 expression), thus demonstrating that bioactive *R_{S}*-GRA is very active in the treatment of this disease.

Additionally, C57BL/6 mice were used in two different sets of experiment, administrated according to an acute model of PD and a sub-acute model of PD, in order to evaluate the neuroprotective effects and the potential therapeutic of the bioactive *R_{S}*-GRA in different phases of the disease.

Endpoints of this research consisted in the evaluation of the main PD markers, such as 1) dopamine transporter (DAT) detection, 2) tyrosine hydroxilase (TH) expression, 3) neuronal apoptotic death assessment (including Bax/Bcl-2 balance and decreasing of dendritic spines), 4) inflammatory cytokine localization (Interleukin-lbeta, IL-1β), 5) exposure to oxidative stress (nitrotyrosine, Nuclear factor-erythroid 2 p45-related factor 2 (Nrf2) and Glial Fibrillary Acidic Protein (GFAP) immunolocalization) and the effects on these parameters of bioactive *R_{S}*-GRA treatment. The achieved results showed that bioactive *R_{S}*-GRA can protect neurons against the neurotoxicity involved in PD via an anti-apoptotic/anti-inflammatory action.

It follows that the bioactivation of *R_{S}*-GRA with Myr enzyme allowed to overcome the above mentioned drawbacks with respect to the synthetic sulforaphane.

In some embodiments, *R_{S}*-Glucoraphanin has a purity higher than 10% weight basis, preferably higher than 50%; in other embodiments, *R_{S}*-Glucoraphanin has a purity higher than 90% weight basis, preferably higher than 95%, wherein *R_{S}*-Glucoraphanin is in the form of a hydrated salt with 1-2 equivalents of water. In a preferred embodiment, *R_{S}*-Glucoraphanin is in the form of a hydrated salt with 1-2 equivalents of water, has a molar extinction coefficient at 225 nm of 6634 1/M*cm and 99% HPLC peak purity.

*R_{S}*-Glucoraphanin can be in the form of an extract of Brassica oleracea L. var. *acephala sabellica.* According to a preferred embodiment, *R_{S}*-Glucoraphanin is a phytochemical and is obtainable by extraction from Brassica oleracea L. var. *acephala sabellica,* said extraction comprising the following steps:
i) grinding the seeds of Brassica oleracea L. var. *acephala sabellica* to a fine powder and defatting with hexane, or grinding the sprouts of Brassica oleracea L. var. *acephala sabellica* to a fine powder,
ii) treating with boiling 70% ethanol,
iii) extracting glucosinolates by using an homogeniser and centrifuging the extract,
iv) isolating *R_{S}*-Glucoraphanin from the glucosinates' extract by one-step anion exchange chromatography, and optionally
v) purifying the isolated *R_{S}*-Glucoraphanin by gel-filtration.

More preferably, seeds are first ground to a fine powder and defatted with hexane. The solvent is removed and the defatted meals are used as starting material. The meals are treated with boiling 70% ethanol in order to quickly deactivate the endogenous enzyme myrosinase, even if temperatures lower than the boiling point can also be used, such as 50-60°C, as well as different ethanol concentrations (%). Glucosinolates are extracted using an Ultraturrax homogeniser at medium speed for 15 min. The resulting homogenate is centrifuged at 17,700×g for 30 min. The isolation of the glucosinolates from the extract was carried out by one-step anion exchange chromatography. Glucosinolate purity is further improved by gel-filtration performed using a XK 26/100 column packed with Sephadex G10 chromatography media (GE Healthcare, Italy), connected to an AKTA-FPLC System (GE Healthcare, Italy). The enzyme myrosinase (Myr, β-thioglucoside glucohydrolase, EC.3.2.1.147) is preferably isolated from seeds of *Sinapis alba L.* as described by Pessina et al. (Pessina, A. et al., 1990. An improved method for the purification of myrosinase and its physicochemical characterization. Arch. Biochem. Biophys. 280, 383-389) with some modification.

Preferably, for the prevention and treatment of neurodegenerative diseases, the composition of the invention is provided so that said *R_{S}*-Glucoraphanin is administered in an amount of 10-500 mg/day and said myrosinase in an amount of 0.4-120U/day. More preferably, said myrosinase is provided as a saline solution at neutral pH.

For the purposes of the present invention, one myrosinase unit (U) is defined as the amount of enzyme able to react with a micro-mole of glucosinolate per min. In fact, *R_{S}*-Glucoraphanin and myrosinase are contacted and reacted, in order to bioactivate *R_{S}*-GRA, before administration, preferably from 10 to 60 minutes before administration. In this regard, it should be noted that the reaction time between *R_{S}*-Glucoraphanin and myrosinase before administration is inversely proportional to the amount of Myr. This means that the higher the reaction time, the lower the amount of Myr.

Preferably, *R_{S}*-Glucoraphanin and myrosinase are contacted and reacted from 15 to 60 minutes, before administration.

In a preferred embodiment, *R_{S}*-Glucoraphanin is administered in an amount of 50-250 mg/day, more preferably said amount is administered twice a day.

The composition of the invention can be administered by oral, parenteral, inhalating, aerosol, spray, transdermal, intravenous, intramuscular, transdermal, subcutaneous, intraperitoneal, intranasal, intrarectal, sublingual or topical route; preferably, by oral, parenteral, or intraperitoneal route; more preferably by oral route.

Optionally, the composition of the invention can comprise also pharmaceutically acceptable carriers.

The term "carrier" refers to a vehicle, excipient, diluent, buffer or adjuvant with which the therapeutic or active ingredient is administered. Any carrier suitable for the form of preparation desired for administration is contemplated for use with the compounds disclosed herein.

The carrier may take a wide variety of forms depending on the form of preparation desired for administration, e.g., oral, intraperitoneal (i.p.) or parenteral (including intravenous). In preparing the compositions for oral dosage form, any of the usual pharmaceutical media may be employed, such as, for example, water, glycols, oils, alcohols, flavoring agents, buffers, preservatives, coloring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as, for example, powders, hard and soft capsules and tablets, with the solid oral preparations being preferred over the liquid preparations.

Preferably, said carrier is a buffered solution at neutral pH (pH 6-8), more preferably a Phosphate-Buffered Saline (PBS) solution at pH 7.2.

In a preferred embodiment, the present invention concerns a composition consisting essentially of *R_{S}*-Glucoraphanin (*R_{S}*-GRA) and myrosinase (Myr), for use as a neuroprotective agent for preventing and treating neurodegenerative diseases. With the expression "consisting essentially of' is meant that *R_{S}*-Glucoraphanin (*R_{S}*-GRA) and myrosinase (Myr) are the only active principles present in the composition having effectiveness against the relevant diseases.

In a more preferred embodiment, the present invention concerns a composition consisting of *R_{S}*-Glucoraphanin (*R_{S}*-GRA), myrosinase (Myr), and pharmaceutically acceptable carriers, for use as a neuroprotective agent for preventing and treating neurodegenerative diseases.

The addition of said carriers to the composition of the invention can be performed according to conventional pharmaceutical compounding techniques.

In another aspect, the present invention concerns a kit for providing the composition for use as above described, comprising:
a) a container comprising *R_{S}*-Glucoraphanin,
b) a container comprising myrosinase, and
c) a leaflet comprising instructions for simultaneous, separate or sequential use of *R_{S}*-Glucoraphanin and myrosinase, in preventing and treating neurodegenerative diseases.

*R_{S}*-Glucoraphanin in container a) can be in solid powdered form or in a buffered solution at neutral pH, i.e. pH 6-8. Most preferably, said buffered solution is a Phosphate-Buffered Saline (PBS) solution at pH 7.2.

Preferably, the container b) comprises a saline solution of myrosinase having neutral pH.

In a particularly preferred embodiment, myrosinase is in a saline solution having neutral pH at a concentration from 5 to 50 U/ml, more preferably, said myrosinase is provided in a saline solution at neutral pH at a concentration from 25 to 35 U/ml.

In a further embodiment, the container a) and the container b) are adjacent compartments of the same delivery system, divided by a breakable septum.

It should be understood that all aspects identified as preferred and advantageous for the composition of the invention are also to be considered similarly preferred and advantageous for the kit and the use thereof.

Working examples of the present invention are hereinafter provided for illustrative and nonlimiting purposes.

### EXAMPLES

### Example 1.

### Evaluation of the anti-inflammatory and anti-apoptotic effects of R_{S}-Glucoraphanin bioactivated with myrosinase in murine sub-acute and acute MPTP-induced Parkinson's Disease (PD)

### Materials and Methods

### Animals

Male C57BL/6 mice (n=50, 4-5 week old, 20-25g weight) were purchased from Harlan (Italy). Animals were housed in individually ventilated cages with food and water *ad libitum.*

The room was maintained at a constant temperature and humidity on a 12h/12h light/dark cycle. Animal care was in compliance with Italian regulations on protection of animals used for experimental and other scientific purposes (D.M. 116192) as well as with European Economic Community regulations (O.J. of E.C. L 358/1 12/18/1986).

Experimental procedures did not cause any significant animal suffering.

### MPTP-induced acute and sub-acute PD

PD was induced by MPTP hydrochloride (Sigma-Aldrich, Italy) injection, a neurotoxin that causes a selective destruction of DA neurons. 1-Methyl-4-phenyl-1,2,3,6-tetrahydropyridine (MPTP)-induced neurotoxicity is the most common experimental model used through which mice show typical neuropathological defects as observed in human PD. MPTP is converted to 1-methyl-4-phenylpyridinium (MPP+) by monoamine oxidase B and it is carried by the dopamine transporter (DAT) into DA cells, causing the block of mitochondrial complex I activity

In this experimental design, two different sets of experiment were performed: the first one to induce an acute (2 injections for MPTP 40mg/kg) Parkinsonian syndrome, while the second one (5 injections for MPTP 20mg/kg) to produce sub-acute symptoms.

### R_{S}-Glucoraphanin and Myrosinase purification

*R_{S}*-Glucoraphanin (*R_{S}*-GRA) was isolated according to the preferred extraction procedure above reported. Briefly, seeds of *Brassica oleracea* L. var. acephala sabellica (Tuscan black kale) supplied by SUBA & UNICO (Longiano, Italy) were grinded to a fine powder and subsequently defatted with hexane. The solvent was removed and the defatted seed meal was treated with boiling 70% ethanol in order to quickly deactivate the endogenous enzyme Myr. *R_{S}*-GRA was extracted using an Ultraturrax homogeniser at medium speed for 15 min. The resulting homogenate was centrifuged at 17,700×g for 30 min. The isolation of the *R_{S}*-GRA from the extract was carried out by one-step anion exchange chromatography, and the purity was further improved by gel-filtration performed using a XK 26/100 column packed with Sephadex G10 chromatography media (GE Healthcare, Italy), connected to an AKTA-FPLC System (GE Healthcare, Italy). Individual fractions were analyzed by HPLC and those containing pure *R_{S}*-GRA were pooled and freeze-dried. *R_{S}*-GRA was characterized by ¹H and ¹³C NMR spectrometry and the purity was assayed by HPLC analysis of the desulfo-derivative according to the ISO 9167-1 method. The purity was 99% (peak purity HPLC) and >95% weight basis. The enzyme Myr was isolated from seeds of *Sinapis alba* L. as described by Pessina et al. with some modification. The specific activity of the stock solution used in the present study was 60 U/mg of soluble protein. The enzymatic activity was 32 U/ml and the solution was stored at 4°C in sterile saline solution at neutral pH until use. One Myr unit was defined as the amount of enzyme able to hydrolyze at pH 6.5 and 37°C 1µmol/min of sinigrin [Abdull Razis, A.F., et al., Up-regulation of cytochrome P450 and phase II enzyme systems in rat precision-cut rat lung slices by the intact glucosinolates, glucoraphanin and glucoerucin. Lung Cancer, 2011. 71(3): p. 298-305].

### Enzyme bio-activation of Rs-GRA and animal treatment

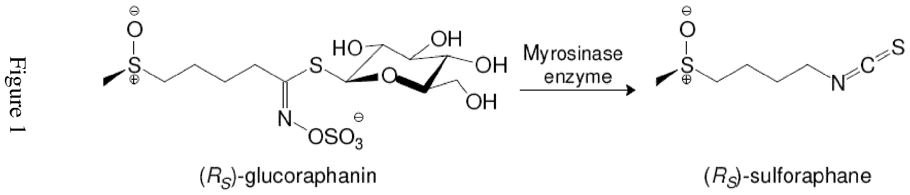

*R_{S}*-GRA (10 mg/kg) was dissolved in PBS solution pH 7.2 and mouse treatment required the enzyme bioactivation of the phytochemical. The action of the Myr enzyme (5 µl/mouse) for 15 min allowed to have a bioactive *R_{S}*-GRA quickly, before the i.p treatment (Figure 1).

### Timetable and experimental animal groups

Mice were randomly divided according to the following scheme:
1. **ACUTE PD group** (n=10): Mice received two injections of MPTP (40 mg/kg, i.p.) 16h apart.
2. **ACUTE PD** + **bioactive *R_{S}*-GRA group** (n=10): Mice were daily pre-treated for 1 week with bioactive *R_{S}*-GRA (10 mg/kg, i.p.), before the two injections of MPTP (40 mg/kg, i.p.) 16h apart. Bioactive *R_{S}*-GRA treatment was protracted until the sacrifice.
3. **SUB-ACUTE PD group** (n=10): Mice received five injections of MPTP (20mg/kg, i.p.) 24h apart.
4. **SUB-ACUTE PD** + **bioactive *R_{S}*-GRA group** (n=10): Mice were daily pre-treated for 1 week with bioactive *R_{S}*-GRA (10mg/kg, i.p.), before the five injections of MPTP (20mg/kg, i.p.) 24h apart. Bioactive *R_{S}*-GRA treatment was protracted until the sacrifice.
5. **NAÏVE group** (n=5): Mice no subjected to MPTP damage or to any injection, sacrificed as control.
6. **Bioactive *R_{S}*-GRA-CONTROL group** (n=5): Mice no subjected to MPTP damage but injected with bioactive *R_{S}*-GRA (10mg/kg), sacrificed as control of drug safety and tolerance as well as to evince eventually behavioral changes on the animals.

Schematically, a plan of the experiment below:

| Days | SUB-ACUTE PD |
|---|---|
| 0-7 | bioactive *R_{S}*-GRA pretreatment (10mg/kg) |
| 8-12 | MPTP (20mg/kg) + bioactive *R_{S}*-GRA (10mg/kg) |
| 13-14 | bioactive *R_{S}*-GRA (10mg/kg) |
| 15 | Sacrifice |

| Days | ACUTE PD |
|---|---|
| 0-7 | bioactive *R_{S}*-GRA pretreatment (10mg/kg) |
| 8-9 | MPTP (40mg/kg) + bioactive *R_{S}*-GRA (10mg/kg) |
| 10-14 | bioactive *R_{S}*-GRA (10mg/kg) |
| 15 | Sacrifice |

### Immunohistochemical (IHC) localization of IL-1β, DAT, nitrotyrosine, Bax, Bcl-2, TH

After the sacrifice, brain tissues were collected and fixed in 10% (w/v) PBS-buffered formaldehyde. Sections (6µm thickness) were prepared from paraffin embedded tissues. After deparaffinization, endogenous peroxidase was quenched with 0.3% (v/v) hydrogen peroxide in 60% (v/v) methanol for 30 min. The sections were permeabilized with 0.1% (w/v) Triton X-100 in PBS for 20 min. Nonspecific adsorption was minimized by incubating the section in 2% (v/v) normal goat serum in PBS for 20 min. Endogenous biotin or avidin binding sites were blocked by sequential incubation for 15 min with avidin-biotin peroxidase complex. Sections were incubated overnight with different antibody:
1) rabbit polyclonal anti-DAT (1:250 in PBS, w/v) (Santa Cruz Biotechnology. INC);
2) rabbit polyclonal anti-TH (1:100 in PBS, w/v) (Santa Cruz Biotechnology. INC);
1) rabbit polyclonal anti-IL-1β (1:100 in PBS, w/v) (Santa Cruz Biotechnology. INC);
2) rabbit polyclonal anti-nitrotyrosine (1:500 in PBS, w/v) (Millipore);
3) rabbit polyclonal anti-NrF2 (1:100 in PBS, w/v) (Santa Cruz Biotechnology. INC);
4) goat polyclonal anti-GFAP (1:50 in PBS, w/v) (Santa Cruz Biotechnology. INC).
5) rabbit polyclonal anti-Bcl-2 (1:100 in PBS, w/v) (Santa Cruz Biotechnology. INC);
6) rabbit polyclonal anti-Bax (1:100 in PBS, w/v) (Santa Cruz Biotechnology. INC).

Sections were washed with PBS, and incubated with secondary antibody. Specific labeling was detected with a biotin-conjugated goat anti-rabbit IgG and avidin-biotin peroxidase. The counter stain was developed with avidin-biotin peroxidase (brown staining) and Mayer's hematoxylin (blue background). A positive staining (brown color) was found in the sections, indicating that the immunoreactions were positive.

To verify the binding specificity for IL-1β, DAT, nitrotyrosine, Bax, Bcl-2, Nrf2 and GFAP some sections were also incubated with only the primary antibody (no secondary) or with only the secondary antibody (no primary). In these situations no positive staining was found in the sections indicating that the immunoreactions were positive in all the experiments carried out.

### Golgi Stain

FD Neurotech kit (FD NeuroTechnologies, Ellicott City, Md, USA) was used for Golgi impregnation of tissue.

Brains were placed directly into solution (A+B) containing mercuric chloride, potassium dichromate and potassium chromate, without rinsing, and remained there for 2 weeks in the dark at room temperature. Forty-eight hours after placing in solution C (4°C), the brains were frozen on dry ice and stored at -70°C until sectioning. Cryostat sections (100µm) were cut at -25°C and mounted onto gelatinized slides. Slides were allowed to dry in the dark, and the rest of the staining process done as previously described [Feng, J., et al., Spinophilin regulates the formation and function of dendritic spines. Proc Natl Acad Sci U S A, 2000. 97(16): p. 9287-92].

Neurons were chosen for the analysis if completely impregnated with Golgi stain and unobscured by other impregnated neurons or precipitant. To perform spine count, the longest dendritic trees visible within the plane of focus, with continuous unbroken dendrites, well impregnated cells and clearly distinguishable from adjacent, were chosen. Spines were counted under oil (X100), using light microscopy (LEICA ICC50 HD), and the entire visible dendritic length measured by Imaging computer program (Leica Application Suite V4.1). Spine density was calculated referring to the length of the dendrite.

### Body weight loss and behavioral tests

The measure of the body weight was daily assessed, and any loss was evaluated as marker of pathology. Body weight changes have been represented as mean for group of loss or increase grams compared to naive mice. At the first day of observation, a value 1 was attributed to naive group, and each increasing/decreasing was considered as a deviation from this baseline. Moreover, each induction of MPTP was followed by the behavioral tests listed below:
- **Open field:** Motor function of the animal is measured by quantified spontaneous activity in the open field. Locomotor activity was monitored for 3 min in an open field, a white Plexiglas box 50 × 50 cm with its floor divided into 16 squares. Four squares were defined as the center and 12 squares along the walls as the periphery. Each mouse was gently placed in the center of the box and activity was scored as a line crossing when a mouse removed all four paws from one square and entered another. Immediately after each test, the apparatus was thoroughly cleaned with cotton pad wetted with 70% ethanol. The line crossings and the time spent in the center were counted and scored. Just for 20mg/kg MPTP-administrated group and the relative bioactive *R_{S}*-GRA-treated group, the test was performed after a latency period of 5min, during which mice were placed on the table and the time elapsing first change of square was noted.
- **Pole test:** The pole test is used to assess basal ganglia related movement disorders in mice. In particular, it is a useful method in mice PD model to measure bradykinesia [Ogawa, N., et al., A simple quantitative bradykinesia test in MPTP-treated mice. Res Commun Chem Pathol Pharmacol, 1985. 50(3): p. 435-41].
   Briefly, after 20mg/kg MPTP-injection and the open field test, animals are placed head-up on top of a vertical wooden pole (diameter 5mm, height 55cm, with rough surface). The base of the pole is placed in a cage filled with bedding material. When placed on the pole, animals orient themselves downward and descend the length of the pole back into the cage. During the behavioral test, the animals was videotaped and timed. The time that mice needed to turn down completely and climbed down and four feet reached the floor was recorded.

### Statistical Analysis

All statistical comparisons of values between two animal groups were carried out by unpaired Student's t-test and multiple group comparisons were conducted by one-way analysis of variance (ANOVA) with Bonferroni test with the Prism software (GraphPad Software, Inc., La Jolla, CA, USA). Data are represented as mean ± S.E.M. In all cases, a P value of <0.05 was considered to be significantly different.

### Results

### Bioactive R_{S}-GRA-treatment avoids body weight changes resulting in sub-acute and acute MPTP-induced PD

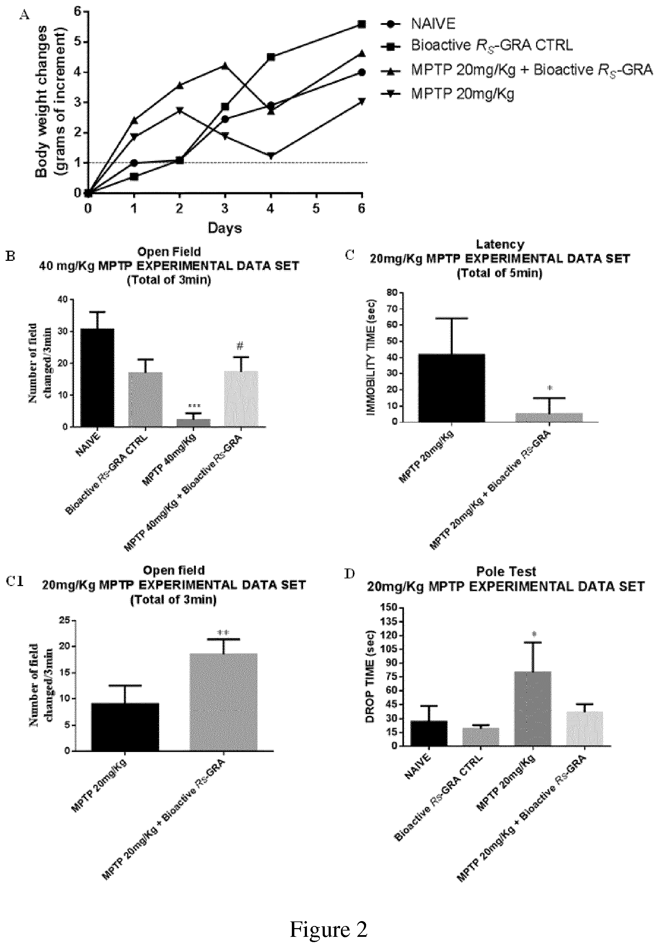

No significant body weight loss was found in acute (40mg/kg MPTP) experimental set when animals were compared with bioactive *R_{S}*-GRA-treated mice and naive group (data not shown). On the contrary, Figure 2A summarizes the results of body weight determination comparing sub-acute 20mg/kg MPTP group, bioactive *R_{S}*-GRA-control group, naive group and MPTP-administrated animals treated with bioactive *R_{S}*-GRA.

Firstly, it should be noted that compared to naive, PD group showed a higher body weight at the beginning of observation time (day 1). Nevertheless, causing the progression of the disease, the body weight of 20 mg/kg MPTP-administrated mice rapidly and continuously decreased up to the end of the experimental period. In particular, after the fourth injection of MPTP, there was a consistent difference among PD group and all other groups.

On the contrary, after PD induction, bioactive *R_{S}*-GRA-treated mice increased their body weight. No sign of any decrease in body weight was detected in bioactive *R_{S}*-GRA-treated control group.

### Motor behavior evidences about bioactive R_{S}-GRA-treatment protective role in sub-acute and acute PD development

A square table divided in sub-squares by a grid was used for open-field test.

For 40mg/kg MPTP-administrated mice, the test was performed without any latency time, since the animals showed a constant behavioral immobility. The results showed that reduced degree of mobility and a little number of changes was noted in acute MPTP-administrated animals, while bioactive *R_{S}*-GRA-treatment after MPTP-administration preserved the mice by the neurological behavioral changes (Figure 2B), also compared to the results of naive and bioactive *R_{S}*-GRA-control group.

For 20 mg/kg MPTP-administrated mice, the test was started by placing the mice at the center of the table to be familiar with the situation for a time of 5 min (latency-time), then the total horizontal ambulatory route and the spatial analysis activity within the period of the following 3 min was measured by observers. Looking at the latency-time, sub-acute MPTP-administration causes an immobility time of around 40 sec, while bioactive *R_{S}*-GRA-treated mice have showed a latency-time around equal to zero.

To confirm these results, immediately after the open field, the same animals were examined for evaluating the degree of PD-related bradykinesia, performing a pole test (Figure 2 C1). 20 mg/kg MPTP-administrated mice treated with bioactive *R_{S}*-GRA did not show bewilderment when placed head-up on top of the vertical wooden pole and compared with the untreated groups. Moreover, pharmacological injection of bioactive *R_{S}*-GRA caused faster drop time, if the results are compared with sub-acute MPTP-group that didn't receive this treatment (Figure 2 C1).

### Bioactive R_{S}-GRA-treatment prevents DAT loss and TH decrease in nigrostriatal neuronal cells

Scientific literature about PD feature and pathway largely shows that the plasma membrane DAT is a reliable marker of presynaptic DA terminal loss. So, each cellular role currently associated with the DAT depends on plasma membrane expression.

One of the first signs of MPTP-induced neurotoxicity is the neuronal damage in the substantia nigra, that causes a decreasing of DAT expression, both in sub-acute and acute PD brain tissue sections.

On the contrary, the neuroprotective action of bioactive *R_{S}*-GRA-treatment in sub-acute and acute MPTP-regime prevented the loss of DA transporter expression in brain section. Sections from naive group or drug control group, incubated with anti-DAT antibody, showed no any reduction in the level of expression of this marker.

The pathophysiology of PD is largely correlated with a decrease in tyrosine TH activity, TH synthesis and TH mRNA in the striatum. Thus, due to its importance as rate limiting enzyme in the conversion of tyrosine into L-DOPA during dopamine synthesis, it was decided to assess the changes in the expression levels of TH. Both 20 mg/kg and 40 mg/kg MPTP-injections followed by bioactive *R_{S}*-GRA-treatment evoked an increased TH expression in striate brain section, that correlated with the return of striatal dopamine. Conversely, brain sections taken from bioactive *R_{S}*-GRA-untreated sub-acute and acute MPTP-administrated mice and incubated with anti-TH antibody, showed negative staining for this marker. An intense positive staining was found in bioactive *R_{S}*-GRA-control group samples.

### Bioactive R_{S}-GRA-treatment inhibits expression of IL-1β, modulating inflammatory pathway

A key role in PD development was attributed to cytokine release, that was associated with a progressive condition of disease in animals MPTP-administrated. In particular, mice expressing a dominant-negative inhibitor of IL-1β-converting enzyme or those deficient in TNF-α or iNOS seem resistant to MPTP-induced neurotoxicity.

The present results showed that brain sections cut from sub-acute and acute MPTP-administrated mice treated with bioactive *R_{S}*-GRA showed a reduced IL-1β IHC localization.

Conversely, sub-acute and acute MPTP-administrated mice that didn't received bioactive *R_{S}*-GRA-treatment have high levels of IL-1β expression, supporting the idea according to which one of the possible mechanism of action of bioactive *R_{S}*-GRA should be an anti-inflammatory role of the molecule.

It should be noted that no any IL-1β positive staining was detected for bioactive *R_{S}*-GRA-treated control mice.

### Bioactive R_{S}-GRA-treatment preserves neuronal cells by oxidative stress after sub-acute and acute MPTP-induced PD: evidences by nitrotyrosine, Nrf2 and GFAP expression

One consequence of upstream inhibition of the inflammatory pathway is the possible blockage of the oxidative stress triggering. Increased nitration of proteins in motor neurons has been identified in patients with neurodegenerative disease.

For this reason, it was chosen as indirectly marker for peroxynitrite formation *in vivo* the IHC detection of nitrotyrosine expression.

Bioactive *R_{S}*-GRA inhibits MPTP-induced oxidant production and oxidative DNA damage in the SNpc.

20 mg/kg and 40 mg/kg MPTP-administrated mice showed significant positive staining for nitrotyrosine. The corresponding bioactive *R_{S}*-GRA-treated animal groups presented a reduced tissue expression for this marker in both sub-acute and acute conditions.

Drug control mice showed no any staining for nitrotyrosine.

Moreover, to confirm the reduced oxidative stress after bioactive *R_{S}*-GRA-treatment, Nrf2 IHC localization was performed.

A negative expression was found in histological slides of subacute and acute MPTP-administrated mice untreated with bioactive *R_{S}*-GRA, that, when given, on the contrary, kept Nrf2 nuclear expression at high levels, preserving tissues by MPTP-damage.

It should be noted that brain sections taken from bioactive *R_{S}*-GRA animals no subjected to MPTP-administration and changes didn't stain after Nrf2 incubation. This was believed normal, considering that animals weren't subjected to any stress or damage (quiet state).

To confirm this data, it was stained brain section with an α-GFAP antibody. As expected in agreement with previous results, sub-acute and acute MPTP-administrated mice exhibited positive immunolocalization for cytosolic GFAP, whereas bioactive *R_{S}*-GRA-treatment reduced the degree of positive staining for GFAP in mice subjected to MPTP-induced sub-acute and acute injury. Bioactive *R_{S}*-GRA-control brain samples section did not stain for GFAP as well.

### Bioactive R_{S}-GRA-treatment protects DA neurons against apoptotic cell death

Brain tissue samples were also collected 8 days after MPTP-administration for IHC detection of Bax and Bcl-2.

Sections cut from bioactive *R_{S}*-GRA-control mice samples did not stain for Bax, whereas tissue MPTP-administrated sub-acute and acute mice exhibited positive immunolocalization. Bioactive *R_{S}*-GRA-treatment reduced the degree of positive staining for Bax in mice subjected to MPTP-induced sub-acute and acute injury.

On the contrary, brain tissue sections cut from bioactive *R_{S}*-GRA-treated control mice demonstrate positive staining for Bcl-2, while in MPTP-administrated sub-acute and acute mice staining for Bcl-2 was absent. Bioactive *R_{S}*-GRA-treatment significantly attenuated the loss of positive staining for Bcl-2 in the samples cut from mice subjected to MPTP-induced sub-acute and acute injury.

### Bioactive R_{S}-GRA-treatment protects DA neurons against impaired synaptic functionality and plasticity

Brain disorders should be associated with abnormal number of dendritic spines and correlated to abnormal neuronal cell function in terms of biochemical signals, in particular Ca²⁺-mediated synaptic plasticity. Quantitative analysis of dendrite spine density was performed through Golgi impregnation in hypothalamic dental nucleus sections. Microscopic images were acquired and following, the number of spines for a total number of 10 fields was obtained.

In the dentate gyrus of MPTP-injured animals in both sub-acute (see mean of dendritic spines/10 fields, Figure 3A) and acute (Figure 3D, see mean of dendritic spines/10 fields, Figure 3A) trend, the number of tree branches resulted significantly reduced. Conversely, bioactive *R_{S}*-GRA-treatment protects the neuronal functional disruption (Figure 3E and F, see mean of dendritic spines/10 fields, Figure 3A), with a dendritic spine count for field that resembling the results obtained for bioactive *R_{S}*-GRA-control and naive groups (Figure 3B, see mean of dendritic spines/10 fields, Figure 3A).

### Discussion

It was demonstrated the neuroprotective power of *R_{S}*-GRA bioactivated with Myr, nearly before the administration time, as a novel important field of action applicable in the prevention and treatment of PD, widely associated both to inflammatory response and oxidative mechanism, as well as to apoptotic pathway and neurodegenerative feature.

The pathological hallmark of idiopathic PD is the loss of DA neurons in the SNpc. Dopamine transporter (DAT) deficiency is an important illness factor in PD, being reduced by 50 to 70% and playing a key role in the occurrence of motor complications. The treatment with bioactive *R_{S}*-GRA represents a beneficial protective factor, into avoiding the molecular deficit of DAT and this data is supported by the evidence that TH, involved into metabolism of dopamine, is preserved by bioactive *R_{S}*-GRA administration as well.

Visibly, this has resulted in a good motor capability, absence of bewilderment, bradykinesia, tremor and of all behavioral symptom of disease.

Despite the mechanism through which PD signs are reduced by bioactive *R_{S}*-GRA is not clarified, without wishing to be bound by any theory, it can be hypothesized that it could modulate the triggering of the inflammatory cascade and the responses to it correlated. Thus, this assumption was confirmed by the investigation of key regulators of 1) inflammatory response 2) oxidative damage and 3) apoptotic pathway.

Inflammation has been implicated in the pathogenesis of PD. In particular, microglial activation, following NFkB translocation during PD, lead to the related release of Cox-2, IL-1β, and Toll-like receptor activation, resulting in increased degeneration of DA neurons of the SNpc.

By the analysis of the inventors of one among these hallmarks, resulted that, effectively, there is a dowregulation of IL-1β in animals treated with bioactive *R_{S}*-GRA, showing that, one of the possible action ways of this molecule is the control of the cytokine production. Other evidences were given by the analysis of stress oxidative degree after bioactive *R_{S}*-GRA treatment.

Oxidative stress has been proposed as a pathogenetic mechanism in PD. One mechanism of oxidative cellular injury is the nitration of protein tyrosine residues, mediated by peroxynitrite, a reaction product of nitric oxide and superoxide radicals. Thus, elevated levels of nitrosative stress have been linked to the pathogenesis of PD. Administration of bioactive *R_{S}*-GRA is correlated to a reduced degree of nitrotyrosine detection, as further proof that the action of this molecule goes through a mechanism powerful enough to stop the damage from nitrosative stress. Moreover, being Nrf2 believed one of the most important transcription factors involved in the protection of the cells by oxidative stress, regulating cytoprotective genes and triggering the activation of the antioxidant glutathione (GSH) pathway, it was decided to verify the expression level of this key regulator.

Thus, in agreement with the current literature, in the present work, the significative Nrf2 expression - found after bioactive *R_{S}*-GRA treatment - seems to play a protective action in MPTP-induced sub-acute and acute PD, decreasing GFAP expression and probably through the mechanism that above mentioned inventors declare related to the glutathione activity.

Finally, it was analyzed the grade of damage related to the activation apoptotic of pathway after PD-induction. Administration of MPTP into two different doses at different times, produces in the animals a different symptomatology, obviously more severe and constant in the acute group and lighter and temporary but, however, according an relapsing-remitting appearance in sub-acute group. So, analyzing the degree of apoptosis in sub-acute and acute MPTP-injected mice, it was found a neuroprotection due to prophylactic pretreatment by bioactive *R_{S}*-GRA, commensurate to the damage power of the toxic molecule.

Interestingly, Bax/Bcl-2 balance isn't altered after bioactive *R_{S}*-GRA-treatment both in sub-acute and acute MPTP-regime. Likewise, further demonstration of neuroprotection and maintenance of the neuronal functionality was the unaffected number of dendritic spines, preserved following the pharmacological treatment.

Taken together, these results show a protective role of bioactive *R_{S}*-GRA against MPTP neurotoxicity in mice, suggesting that it could be an advantageous treatment in early, not advanced or chronic phases of PD.

The DA neurodegeneration in PD caused by a cascade of events involving different inflammatory mediators and downstream cascade both due to radicalic species and apoptotic intermediates is affected by bioactive *R_{S}*-GRA-treatment, the benefits of which can be obtained either in the form of readily available dietary supplement or as a therapeutic agent safe and well tolerated.

### Example 2.

### Evaluation of the protective role of (R_{S})-Glucoraphanin bioactivated with myrosinase in an experimental model of multiple sclerosis (MS)

### Materials and Methods

### Animals

C57BL/6 adult male mice (20-25 g, Harlan Nossan, Milan, Italy) were used for all studies. Mice were housed in stainless steel cages and maintained under 12h/12h light/dark cycle at 21 ± 1°C and 50 ± 5% humidity. The animals were acclimated to their environment for 1 week and food and water were given *ad libitum.* Animal care was in compliance with Italian regulations on protection of animals used for experimental and other scientific purposes (D.M. 116192) as well as with the EEC regulations (O.J: of E.C.L 358/1 12/18/1986).

Experimental procedures did not cause any significant animal suffering.

### Reagents

The Myelin Oligodendrocyte Glycoprotein peptide (MOG)35-55 (MEVGWYRSPFSRVVHLYRNGK; Auspep) was synthesized and purified by Cambridge Research Biochemicals (Billingham, UK).

Complete Freund's Adjuvant (CFA) containing *Mycobacterium tubercolosis* H37Ra was purchased from Difco Laboratories (Sparks, MD, USA), while *Bordetella pertussis* toxin was from Sigma-Aldrich Company Ltd. (Milan, Italy).

Unless otherwise stated, all compounds were obtained from Sigma-Aldrich Company Ltd. All other chemicals were of the highest commercial grade available.

### Induction of Experimental Autoimmune Encephalomyelitis (EAE)

After anesthesia, mice were immunized subcutaneously with 300µl/flank of the emulsion consisting of 300 µg of MOG₃₃₋₅₅ in PBS combined with an equal volume of CFA containing 300 µg heat-killed *M. Tubercolosis* H37Ra. After emulsion-injection, animals were immunizated with an injection of 100 µl of *B. Pertussis* toxin (500 ng/100 µl, i.p), repeated 48 hours later. The disease followed a course of progressive degeneration, with visible signs of pathology consisting of flaccidity of the tail and loss of motion of the hind legs.

### R_{S}-Glucoraphanin and Myrosinase purification, enzyme bioactivation of Rs-GRA

*R_{S}*-Glucoraphanin and Myr were isolated according to a procedure of Example 1. Enzyme bio-activation of *R_{S}*-Glucoraphanin to have a bioactive *R_{S}*-GRA quickly was carried out according to a procedure of Example 1.

### Experimental design

Mice were randomly allocated into the following groups (N=50 total animals):
- **EAE + bioactive *R_{S}*-GRA group (N=20):** mice subjected to EAE were treated with bioactive *R_{S}*-GRA (10 mg/kg + 5 µl/mouse myrosinase, i.p). Bioactive *R_{S}*-GRA was daily administrated 1week before EAE-induction and, after immunization the treatment was daily protracted until the sacrifice;
- **EAE group (N=20):** mice subjected to EAE that didn't receive bioactive *R_{S}*-GRA;
- **Sham group (N=5):** mice that received vehicle (saline) in place of MOG;
- **Bioactive *R_{S}*-GRA-Control group (N=5):** mice that received vehicle (saline) in place of MOG and treated with bioactive *R_{S}*-GRA (10 mg/kg + 5 µl/mouse myrosinase, i.p.).

The experiment provided a housing period of duration of 7 days followed by a period of pretreatment with bioactive *R_{S}*-GRA via i.p. injection once a day for 7 days. On the fifteenth day, the disease was induced according to the following experimental procedure. In the experimental group EAE + bioactive *R_{S}*-GRA, the post-drug treatment was continued for a further 7 days after induction of the disease until the twenty-first day.

At the end of the experiment the animals were sacrificed, and spinal cord tissues were harvested and processed, in order to evaluate parameters of disease.

### Body weight and clinical score

Mice were observed daily for signs of EAE. Clinical score was evaluated using a standardized scoring system [Rodrigues DH, et al., Absence of PI3Kgamma leads to increased leukocyte apoptosis and diminished severity of experimental autoimmune encephalomyelitis. J Neuroimmunol 2010;222:90-94]. Briefly, clinical signs were scored as follows: 0= no signs; 1= partial flaccid tail; 2= complete flaccid tail; 3= hind limb hypotonia; 4= partial hind limb paralysis; 5= complete hind limb paralysis; 6= moribund or dead animal.

In addition, the measure of the body weight was daily assessed, and any loss was evaluated as marker of pathology. Body weight changes have been represented for each group as mean of loss or increase grams compared to sham mice. At the first day of observation, a value 1 was attributed to sham group, and each increasing/decreasing was considered as a deviation from this baseline (Figure 4).

### Western Blot Analysis for IL-1β, JNK, IkB-a, NF-kB, Bax and Caspase 3

All the extraction procedures were performed on ice using ice-cold reagents. In brief, spinal cord tissues were suspended in extraction buffer containing 0.32 M sucrose, 10 mM Tris-HCl, pH 7.4, 1 mM EGTA, 2 mM EDTA, 5 mM NaN₃, 10 mM 2-mercaptoethanol, 50 mM NaF, protease inhibitor tablets (Roche), and they were homogenized at the highest setting for 2 min. The homogenates were chilled on ice for 15 min and then centrifuged at 1000g for 10 min at 4°C, and the supernatant (cytosol + membrane extract from spinal cord tissue) was collected to evaluate content of IL-1β, JNK, IkB-α and Bax.

The pellets were suspended in the supplied complete lysis buffer containing 1% Triton X-100, 150 mM NaCl, 10 mM Tris-HCl, pH 7.4, 1 mM EGTA, 1 mM EDTA protease inhibitors tablets (Roche), and then they were centrifuged for 30 min at 15,000g at 4°C, and the supernatant (nuclear extract) was collected to evaluate the content of NF-kB, and Caspase 3.

Supernatants were stored at -80°C until use. Protein concentration in homogenate was estimated by the Bio-Rad Protein Assay (Bio-Rad) using BSA as standard, and 50 µg of cytosol and nuclear extract from each sample was analysed.

Proteins were separated on sodium dodecyl sulfate-polyacrylamide minigels and transferred onto nitrocellulose membranes (Protran nitrocellulose transfer membrane; Whatman Schleicher and Schuell, Dassel, Germany), blocked with phosphate-buffered saline (PBS) containing 5% nonfat dried milk for 45 min at room temperature, and subsequently probed at 4°C overnight with specific antibodies for IL-1β (1:500 Santa Cruz Biotechnology, Inc), JNK (1:500 Santa Cruz Biotechnology, Inc), IkB-α (1:500 Santa Cruz Biotechnology, Inc), Bax (1:500 Santa Cruz Biotechnology, Inc), NF-kB p65 (1:1000; Santa Cruz Biotechnology), and Caspase 3 (1:1000; Cell Signaling), in 1x PBS, 5% (w/v) non fat dried milk, 0.1% Tween-20 (PMT).

Membranes were incubated with peroxidase-conjugated bovine anti-mouse IgG secondary antibody or peroxidase-conjugated goat anti-rabbit IgG (1:2000, Jackson ImmunoResearch, West Grove, PA) for 1 h at room temperature. To ascertain that blots were loaded with equal amounts of proteic lysates, they were also incubated with antibody for β-Actin protein (1:1000 Santa Cruz Biotechnology, Inc) and α-tubulin (1:250 Santa Cruz Biotechnology, Inc).

The relative expression of the protein bands of IL-1β (-17 kDa), JNK (-46 kDa), Bax (-23 kDa), IkB-a (-37 kDa), NF-kB p65 (-65 kDa), Caspase 3 (-35 kDa), was visualized using an enhanced chemiluminescence system (SuperSignal West Pico Chemioluminescent). The protein bands were scanned and quantitated with ChemiDoc™ MP System (Bio-Rad) and a computer program (ImageJ).

### Statistical evaluation

Prism software (GraphPad software) was used to run all the tests. The results were analyzed by one-way ANOVA followed by a Bonferroni post hoc test for multiple comparisons. A p value of < 0.05 was considered to be statistically significant. Results are expressed as the mean ± S.E.M. of n experiments.

### Results

### Body weight loss as sign of disease

Studies on animal models of EAE have demonstrated that the acute phase of the disease coincides with weight loss, probably due to anorexia and deficient fluid uptake. Weight measurement of immunized mice correlated with the severity of the clinical score and showed a significantly reduced weight loss in EAE mice compared to sham mice. With the progress of the disease, it has been seen that the body weight of EAE mice rapidly and continuously decreased up to the end of the experimental period. Indeed, there is a consistent difference among EAE group and all other groups. On the contrary, after EAE induction, significative body weight gain was found in EAE + bioactive *R_{S}*-GRA group and in bioactive *R_{S}*-GRA-control mice compared with EAE group and sham group (Figure 4).

### Bioactive R_{S}-GRA modulates IL-1β levels

To test whether bioactive *R_{S}*-GRA modulates the inflammatory process through the regulation of secretion of pro-inflammatory cytokines, it was analyzed spinal cord tissue levels of IL-1β. A basal levels of IL-1β production was found in spinal cord samples collected from EAE mice 7 days after EAE induction (Figure 5), while spinal cord levels of IL-1β were attenuated by administration of bioactive *R_{S}*-GRA (Figure 5).

### Effect of bioactive R_{S}-GRA on JNK, IkB-α degradation and NF-kB p65

To investigate the cellular mechanisms whereby treatment with bioactive *R_{S}*-GRA attenuates the development of EAE, c-Jun N-terminal protein kinase (JNK) and nuclear NF-kB expression were evaluated by western blot analysis in spinal cord tissue. Basal expression of JNK was detected in the spinal cord samples from sham animals (Figure 6A), whereas JNK levels were substantially increased in EAE mice (Figure 6A). Bioactive *R_{S}*-GRA treatment prevented the EAE-induced JNK expression (Figure 6A). In addition, NF-kB p65 levels in the nuclear fractions from spinal cord tissue were significantly increased at 7 days after EAE compared to the sham mice (Figure 6B). Bioactive *R_{S}*-GRA treatment reduced the levels of NF-kB p65 (Figure 6B). Also, it was evaluated IkB-a degradation and a basal level of IkB-a was detected both in the spinal cord samples from EAE animals (Figure 6C) and in sham mice. Bioactive *R_{S}*-GRA administration prevented the EAE-induced IkB-a degradation (Figure 6C).

### Effect of bioactive R_{S}-GRA on Bax and Caspase 3 expression

At 7 days after EAE, the appareance of proapoptic protein, Bax, in spinal cord homogenates was investigated by Western blot. Bax levels were appreciably increased in spinal cord samples taken from mice subjected to EAE (Figure 7A). Conversely, bioactive *R_{S}*-GRA treatment prevented the EAE-induced Bax expression (Figure 7A). Sequential activation of caspase plays a central role in the execution-phase of cell apoptosis, so that by Western Blot analysis, it was evaluated the activation of Caspase 3. Caspase 3 levels were appreciably increased in the spinal cord from mice subjected to EAE (Figure 7B). On the contrary, treatment with bioactive *R_{S}*-GRA attenuated the EAE-induced Caspase 3 expression (Figure 7B).

### Discussion

It was demonstrated the neuroprotective power of *R_{S}*-GRA bioactivated with Myr, as a novel important field of action applicable in the prevention and treatment of MS, which is an inflammatory demyelinating disease of the CNS, culminating in progressive neurological deterioration. Like many other autoimmune diseases, it is initiated by an uncontrolled T cell response to autoantigenes presented in the context of class II major histocompatibility complex (MHC) molecules of antigen presenting cells (APCs). The most widely accepted hypothesis suggests a dialogue mediated by T cell receptors (TCR) on CD4⁺ T lymphocytes. These interactions between active CD4⁺ T cells and myelin antigens apparently provoke a massive destructive inflammatory response and promotes continuing proliferation of T and B cells and macrophage activation, which sustains secretion of inflammatory mediators, e.g., cytokines/chemokines.

Astrocytes are the major glial cell within the CNS and have a number of important physiological properties related to CNS homeostasis. Bioactive *R_{S}*-GRA can mediate the astroglial response during EAE by modulation of proinflammatory cytokines, such as IL-1β, that as many other inflammatory mediators, are controlled by NF-kB. Here, it was demonstrated that therapeutic effects of bioactive *R_{S}*-GRA can modulate inflammatory pathway during EAE. It is well known that under normal conditions, NF-kB is present within the cytoplasm in an inactive state, bound to its inhibitory protein IkB-a. In response to a wide range of stimuli including oxidative stress, infection, hypoxia, extracellular signals, and inflammation, IkB-a is phosphorylated by the enzyme IkB-a kinase. Once liberated from its inhibitory protein, NF-kB translocates to the nucleus, where it modulates the transcription of a number of proinflammatory genes. The analysis above demonstrated that bioactive *R_{S}*-GRA treatment inhibits the IkB-a degradation as well as the NF-kB activation. A direct consequence of the inhibitory effect of bioactive *R_{S}*-GRA on NF-kB activation is the reduction of proinflammatory mediators production under its control, such as IL-1β. This cytokine is produced by activated macrophages as a proprotein, which is proteolytically processed to its active form by caspase 1. IL-1β plays an important role in host protection against infections but can also promote tissue damage in chronic inflammatory diseases. Moreover IL-1β is involved in a variety of cellular activities, including cell proliferation, differentiation, and apoptosis.

During EAE, IL-1β was demonstrated fundamental in the pathogenesis of inflammatory progression implicated in EAE, by activation of T lymphocytes and stimulation of the production of other cytokines in turn.

It is hereby clearly confirmed an increase in IL-1β during EAE. On the contrary, attenuated expression of IL-1β was observed in mice which received bioactive *R_{S}*-GRA as previously demonstrated. A dowregulation of IL-1β in animals treated with bioactive *R_{S}*-GRA showed that one of the possible action ways of this treatment is the control of the cytokine production. Therefore, it was shown that bioactive *R_{S}*-GRA administration is able to produce substantial reduction of inflammatory events associated with EAE.

Furthermore, once translocated in the nucleus NF-kB activates the transcription of genes involved in the progression of the inflammatory pathway, such as JNK.

JNK is a subfamily of the mitogen activated protein kinase (MAPK) superfamily. JNK was originally identified by its ability to specifically phosphorylate the transcription factor c-Jun on its N-terminal transactivation domain at two serine residues, Ser63 and Ser73. Kinases of the JNK group of MAPKs are primarily activated by proinflammatory cytokines, such as IL-1β and TNF-α, and stress stimuli such as UV radiation, pH changes, hypoxia, and genotoxic and oxidative stress. Activation of JNK in response to these stimuli has been linked to several biological responses, including proliferation, differentiation and apoptosis. In normal cells, the activation of JNK is an event that determine the activation of pro-apoptotic proteins of the Bcl-2 family, which cause an induction of the release of cytochrome c from mitochondria.

This study has demonstrated that the claimed pharmacological treatment with bioactive *R_{S}*-GRA prevents the EAE-induced JNK expression, being that JNK levels substantially decreased in EAE-injected mice. It is conceivable a mechanism mediated by the upstream inhibition of NF-κB p65 translocation, and probably the inhibition of JNK phosphorylation.

The results obtained from analysis of all these protein parameters led the inventors to investigate the effect of treatment with bioactive *R_{S}*-GRA on the apoptosis degree after EAE induction.

Apoptosis is a natural form of cell death, which can be induced by an "intrinsic" mitochondria mediated pathway. This pathway causes activation of caspases (in particular caspase 3) which, by cleavage of cellular substrates, leads to programmed cell death. Caspase 3 is a key regulator of apoptosis, essential for some of the characteristic changes in cell morphology and in some biochemical events associated with the execution and completion of this process. Since cleaved caspase-3 is considered as marker of apoptosis, it was evaluated the cleaved caspase-3 expression by Western blot analysis. It was found that EAE causes significant increase of cleaved caspase-3 when compared with sham animals, whereas bioactive *R_{S}*-GRA treatment decreased the level of cleaved caspase-3.

Another important pro-apoptotic factor, playing a pivotal role in developmental cell death and in CNS injury is Bax. For this reason, it was detected in EAE proapoptotic transcriptional changes an upregulation of Bax. In particular, it was shown that treatment with bioactive *R_{S}*-GRA reduced Bax expression, leading to a reduction of the proapoptotic pathway activation. This finding suggests that the neuroprotective effect of bioactive *R_{S}*-GRA is associated with apoptosis regulation.

To sum up, these results clearly showed that bioactive *R_{S}*-GRA can be used for treatment of MS, because of its neuroprotective role through a mechanism that involves both the modulation of the inflammatory pathways, and the reduction of the activation of cell death by apoptosis.

### Example 3.

### Evaluation of the effects of R_{S}-Glucoraphanin bioactivated with myrosinase on the tight junction dysfunction in a mouse model of restraint stress

### Materials and Methods

### Animals

CD mice (4-5 weeks old, 20-22 g) were purchased from Harlan (Italy). Animals were housed in a controlled environment and provided with standard rodent chow and water *ad libitum.*

Animal care was in compliance with Italian regulations on protection of animals used for experimental and other scientific purposes (D.M. 116192) as well as with European Economic Community regulations (O.J. of E.C. L 358/1 12/18/1986).

Experimental procedures did not cause any significant animal suffering.

### R_{S}-Glucoraphanin and Myrosinase purification

*R_{S}*-Glucoraphanin and Myr were isolated according to a procedure of Example 1.

### Enzyme bio-activation of R_{S}-Glucoraphanin and animal treatment

A lyophilized *R_{S}*-Glucoraphanin was dissolved in PBS solution pH 7.2 at room temperature to administrate a final concentration of 10 mg/kg. Mouse treatment required the enzyme bioactivation of the phytochemical. The action of the Myr enzyme (5 µl/mouse) for 15 min allowed having a bioactive *R_{S}*-GRA quickly, before the i.p. treatment.

### Experimental protocol

Mice (n = 45) were randomly divided into three groups:
- sham group: animals did not suffer restraint stress: they have been just briefly anesthetized as the other groups and received an equal volume of saline solution via i.p. as treatment;
- stressed mice group: animal body was immobilized with a tape on a table for 150 min at the four extremities;
- stressed mice + bioactive *R_{S}*-GRA group: stressed mice were pretreated 1 h before the immobilization with a single dose of bioactive *R_{S}*-GRA, obtained as above described.

Before the induction of restraint stress, all animals were anesthetized with isoflurane. At the end of the immobilization-time they were sacrificed with an intraperitoneal injection (i.p.) of Tanax (5 ml/kg body weight). Mice were fasted 24 h before the induction of stress. Since fecal pellet output is known to increase under stress conditions, the feces were collected to evaluate the stress effect during the stress period. At the end of the observation-time, mice were sacrificed and the stomach and cerebral hemispheres were collected to perform histological evaluation, immunohistochemistry (IHC) and expression analysis by western blotting.

### Albumin Evan's blue determination

Ten animals for each group were used to assess BBB permeability, quantitatively evaluated by measuring the amount of extravasated Evan's blue dye in brain tissue according to a procedure described by Hawkins BT et al. ('Fluorescence imaging of blood-brain barrier disruption'. Journal of neuroscience methods. 2006;151:262-7).

The animals were immobilized for 150 min, and 60 min before the end of the immobilization, Evan's blue (2%, containing 18 g/l bovine serum albumin 4 ml/kg) was injected in mice into the left femoral vein over 2 min; in order to be able to circulate for 60 min. At the end of the immobilization time, animals were sacrificed and brains were sampled. Brains were weighted and homogenized in fivefold volume of 50% trichloroacetic acid solution. The supernatants were obtained by centrifugation (10 min, 10,000 ×g, at 4 °C). Evan's blue extravasation was quantified in the supernatants by measuring the fluorescence (excitation at 620 nm and emission at 680 nm). The amount of extravasated Evan's blue dye was expressed as nanogram per gram of brain tissue.

### Histological evaluation

After fixation for one week at room temperature in 10% (w/v) PBS buffered formaldehyde, gastric mucosa and hemisphere samples were dehydrated in graded ethanol and embedded in Paraplast (Sherwood Medical). Thereafter, 7-µm sections were deparaffinized with xylene, stained with hematoxylin and observed in a microscope (LEICA DM 2000 combined with LEICA ICC50 HD camera).

### IHC localization of claudin-1, claudin-3, ZO-1, iNOS, nitrotyrosine, NF-kBp65, IkB-alpha, TNF-α, Il-1β, 11-10, GFAP and Nrf2

After deparaffinization with xylene, sections of hemisphere samples were hydrated in graded ethanol. For detection, sliceswere treated with protease (type XIV, Sigma-Aldrich Company Ltd.,Milan, Italy; 2 mg/ml) for 10 min at 37 °C. Detection of claudin-1, claudin-3, NF-kBp65, IkB-a, iNOS, nitrotyrosine, TNF-α, IL-1β, IL-10, Nrf-2 and GFAP was carried out after boiling in citrate buffer, 0.01 MpH 6 for 4 min. Endogenous peroxidase was quenched with 0.3% (v/v) hydrogen peroxide in 60% (v/v) methanol for 30 min. Nonspecific adsorption was minimized by incubating the section in 2% (v/v) normal goat serum in PBS for 20 min. Sections were incubated overnight with:
- rabbit polyclonal anti-claudin-1 (Novus Biologicals,Milan, Italy, 1:100 in PBS);
- rabbit polyclonal anti-claudin-3 (Novus Biologicals, 1:100 in PBS);
- guinea pig polyclonal anti-ZO-1 (Novus Biologicals, 1:100 in PBS);
- mouse monoclonal anti-iNOS (Cell Signaling Technology, Inc., Danvers, MA, 1:100 in PBS);
- rabbit polyclonal anti-nitrotyrosine (Millipore, Milan, Italy, 1:1000 in PBS);
- mouse monoclonal NF-kBp65 (Cell Signaling Technology, 1:100 in PBS);
- rabbit polyclonal anti-IkB-alpha (Santa Cruz Biotechnology, Inc., Santa Cruz, CA, USA, 1:100 in PBS);
- rabbit polyclonal anti-TNF-a(Cell Signaling Technology, 1:100 in PBS);
- rabbit polyclonal anti-IL-1β (Santa Cruz Biotechnology, Inc., 1:50 in PBS);
- rat monoclonal anti-IL10 (Santa Cruz Biotechnology, Inc., 1:50 in PBS);
- rabbit polyclonal Nrf2 (Santa Cruz Biotechnology, Inc., 1:50 in PBS);
- mouse monoclonal GFAP (Santa Cruz Biotechnology, Inc., 1:100 in PBS).

Endogenous biotin or avidin binding sites were blocked by sequential incubation for 15 min with biotin and avidin (DBA, Milan, Italy), respectively. Sections were washed with PBS and incubated with secondary antibody. Specific labeling was detected with a biotin-conjugated goat antirabbit IgG and avidin-biotin peroxidase complex (Vectastain ABC kit, VECTOR). The counterstain was developed with diaminobenzidine (brown color) and hematoxylin (blue background). To verify the binding specificity, some sections were also incubated with only the primary antibody (no secondary) or with only the secondary antibody (no primary). In these situations, no positive staining was found in the sections, indicating that the immunoreaction was positive in all the experiments carried out. All sections were obtained using light microscopy (LEICA DM 2000 combined with LEICA ICC50 HD camera) and studied via an Imaging computer program (Leica Application Suite V4.1).

### Western blotting analysis for caspase 3

Extraction procedure was performed on ice using ice-cold reagents. In brief, brain tissues were disrupted by homogenization in extraction buffer containing 0.32 M sucrose, 10 mM Tris-HCl, pH 7.4, 1 mM EGTA, 2 mM EDTA, 5 mM NaN3, 10mM 2-mercaptoethanol, 50mM NaF, and protease inhibitor tablets (Roche Applied Science, Monza (MB), Italy) for 2 min. The homogenates were chilled on ice for 15 min and then centrifuged at 1000 ×*g* for 10 min at 4°C. The pellets were suspended in the supplied complete lysis buffer containing 1% Triton X-100, 150 mM NaCl, 10 mM Tris-HCl, pH 7.4, 1 mM EGTA, 1 mM EDTA protease inhibitors tablets (Roche Applied Science), and then they were centrifuged for 30 min at 15,000 ×g at 4 °C. The supernatant (nuclear extract) was collected and stored at -80°C to evaluate the content of caspase-3. Protein concentration in homogenate was estimated by the Bio-Rad Protein Assay (Bio-Rad, Segrate, Milan, Italy) using BSA as standard, and 50 µg of nuclear extract from each sample was analyzed. Proteins were separated on SDS-PAGE electrophoresis gel and transferred onto PVDFmembrane (Protran nitrocellulose transfer membrane; Whatman Schleicher and Schuell, Dassel, Germany). Membrane was blocked with PBS containing 5% nonfat dried milk for 45 min at room temperature, and subsequently probed at 4 °C overnight with a specific antibody for caspase-3 (1:1000; Cell Signaling Technology, Inc.) in 1× PBS, 5% (w/v) nonfat dried milk, and 0.1% Tween-20 (PMT). Membrane was incubated with peroxidase conjugated goat anti-rabbit IgG (1:2000, Jackson ImmunoResearch, West Grove, PA) for 1 h at room temperature. To ascertain that blot was loaded with equal amounts of proteic lysates, it was also incubated in the presence of the antibody against alpha-Tubulin (1:250 Santa Cruz Biotechnology, Inc.). The relative expression of the protein bands of caspase-3 (-35 kDa; 17-19 kDa when cleaved) and alpha-Tubulin (55 kDa) were visualized using an enhanced chemiluminescence system (SuperSignal West Pico Chemiluminescent). The protein bands were scanned and quantified with ChemiDoc™ MP System (Bio-Rad).

### Statistical evaluation

Prism software (GraphPad software) was used to run all the tests. The results were analyzed by one-way ANOVA followed by a Bonferroni post hoc test for multiple comparisons and by unpaired Student's t-test. A P value of <0.05 was considered to be statistically significant. Results are expressed as the mean ± S.E.M. of n experiments.

### Results

### Stress parameter

Fecal pellet output collected during the immobilization period was significantly higher than non-stressed mice, thus indicating effective stress induction (P value < 0.0001). Moreover, the treatment with bioactive *R_{S}*-GRA considerably decreased fecal pellet production in stressed mice (vs. non-treated stressed mice, P value < 0.0001) (Fig. 8A).

### Evan's blue extravasation corroborate neuroprotective effects on BBB mediated by bioactive R_{S}-GRA treatment

To verify BBB alterations following the induction of restraint stress, mice were injected with Evan's blue, markers of BBB penetration (Fig. 8B). Significant differences were found between experimental groups, since stressed mice have shown severe levels of extravasation, as a marker of BBB modified permeation, while a significant decrease in Evan's blue penetration in the brain of mice administered with bioactive *R_{S}*-GRA was observed.

### Bioactive R_{S}-GRA treatment protects animals by gastric alteration due to restraint stress

The stressed mice group presented some erosions of gastric mucosae, consisting of damage at the level of the apical barrier zone (Fig. 9) as well as inflammatory cells in the sub-mucosae area (lymphocytes and neutrophils). These data confirmed that stressed mice suffered a stress event. In contrast, sections from bioactive *R_{S}*-GRA-treated mice revealed a normal integrity of marginal gastric mucosae, as seen in the sham group. Bioactive *R_{S}*-GRA treatment restores the barrier architecture In order to evaluate the BBB integrity following the induction of restraint stress, it was investigated the claudin-1 (Fig. 9), claudin-3 (Fig. 10) and ZO-1 (Fig. 11) localization in the BBB vascular endothelium by IHC assay. Sections obtained from sham mice showed positive staining and normal distribution for claudin-1, claudin-3 and ZO-1 (Figs. 9, 10, 11), whereas the signals were absent in the sections obtained from mice sacrificed immediately after 150 min of immobilization stress (Figs. Figs. 9, 10, 11). Bioactive *R_{S}*-GRA treatment normalized the positive staining for claudin-1, claudin-3 and ZO-1 in the brain endothelium of stressed mice (Figs. 9, 10, 11).

### Bioactive R_{S}-GRA modulates expression of iNOS

To determine the role of nitric oxide (NO) produced during restraint stress, iNOS expressionwas evaluated by IHC analysis. Looking at the results obtained from sham animals, brain sections as well as slices cut showing vascular endothelium did not stain for iNOS, whereas brain sections obtained from mice subjected to restraint stress exhibited positive staining for iNOS both at the level of the brain and of the vascular endothelium. Bioactive *R_{S}*-GRA treatment reduced the degree of positive staining for iNOS in different districts. Densitometric analysis is provided in Fig. 12.

### Effect of bioactive R_{S}-GRA on nitrotyrosine formation

Nitrotyrosine, a specific marker of nitrosative stress, was measured by IHC analysis in the brain sections to determine the localization of "peroxynitrite formation" and/or other nitrogen derivates produced during restraint stress. Brain sections as well as slices cut at the vascular endothelium level from sham mice did not stain for nitrotyrosine, whereas sections obtained from stressed mice exhibited positive staining for nitrotyrosine, both at the level of the brain and of the vascular endothelium. Bioactive *R_{S}*-GRA treatment of mice subjected to stress reduced the degree of positive staining for nitrotyrosine in the brain, particularly at the level of the vascular endothelium. Densitometric analysis is provided in Fig. 13.

### Protective effect of bioactive R_{S}-GRA seems to be achieved by the attenuation of NF-kB activity and IkB-alpha degradation

NF-kB was sensible to ROS levels. To demonstrate the involvement of NF-kB in the stress-induced TJ changes, it was evaluated nuclear NF-kB activation and IkB-alpha degradation in several brain districts by IHC assay. NF-kBp65 IHC staining was significantly positive in the sections from stressed mice. Especially, NF-kBp65 was principally localized in the nuclei of the brain cell tissue, the vascular endothelial cells in the ependymal area, BBB vascular endothelial cells and of the hippocampal cells (data not shown). In contrast, the same sections obtained from bioactive *R_{S}*-GRA-treated mice showed a reduced degree of positive staining for NF-kBp65. Densitometric analysis is provided in Fig. 14. A negative staining for IkB-alpha was obtained in the same brain districts from stressed mice when compared to sections from treated mice in which a cytoplasmic localization of IkB-alpha was observed. These data revealed that bioactive *R_{S}*-GRA treatment prevented the stress induced IkB-alpha degradation.

### Bioactive R_{S}-GRA modulate the expression of TNF-α, IL-1β and IL-10

To test whether bioactive *R_{S}*-GRA may modulate the inflammatory process through the regulation of pro-inflammatory and anti-inflammatory cytokine secretion, it was analyzed the brain levels of TNF-α (Fig. 15), IL-1β (Fig. 16) and IL-10 (Fig. 17) by IHC assay in several brain districts. After 150 min of immobilization, it was observed in stressed mice a positive staining both for TNF-α and IL-1β when compared with results from sham mice. The treatment with bioactive *R_{S}*-GRA reduced the degree of positive staining for TNF-α and IL-1β. Densitometric analysis is provided in Figs. 15 and 16. About IL-10 IHC staining, in sham animals, a basal negative staining was showed, quantified to around zero. This is because animals weren't subjected to inflammatory and/or damaging conditions that cause the release of protective cytokines. Also, IHC analysis from stressed mice showed a negative staining for IL-10 of brain tissue and vascular endothelium brain sections. In contrast, brain tissue and vascular endothelium brain section from bioactive *R_{S}*-GRA-treated mice revealed a positive staining for IL-10. Densitometric analysis is provided in Fig. 17.

### Effects of bioactive R_{S}-GRA on Nrf2 activation and nuclear translocation

GLs may exert their cytoprotective effects by the ability to induce expression of several enzymes via the Keap1/Nrf2/ARE pathway. IHC analysis showed a negative staining for Nrf2 in the brain tissue, ependymal cells, BBB arteriolar endothelial cells (see densitometric analysis in Fig. 18) and in the hippocampal cells (data not shown) from sections of stressed mice. In contrast, the same sections obtained from bioactive *R_{S}*-GRA-treated mice revealed a positive staining for Nrf2 at the level of the nucleus of the respective districts (see densitometric analysis in Fig. 18).

### Bioactive R_{S}-GRA treatment modulates GFAP expression in several brain districts

GFAP is expressed by astrocytes and it is involved in many important CNS processes, including cell communication and the functioning of the BBB barrier. Since GFAP has been considered a marker of astrocyte activity, it was evaluated the expression changes of GFAP following restraint stress input by using IHC analysis. It was observed a marked positive staining for GFAP in the section from stressed mice, in particular at the level of the capillary in the brain tissue, the BBB arteriolar endothelium as well as of the astroglia (see densitometric analysis in Fig. 19). In contrast, a reduction of GFAP staining was evident in sections of each brain area from stressed mice treated with bioactive *R_{S}*-GRA (see densitometric analysis in Fig. 19).

### Effect of bioactive R_{S}-GRA on caspase 3 expression

It was evaluated whether caspase 3 signaling was involved in the stress-induced damage by western blotting. Caspase-3 levels were appreciably increased in the brain from mice that suffered the stress. On the contrary, treatment with bioactive *R_{S}*-GRA decreased the stress induced caspase-3 expression (see densitometric analysis in Fig. 20).

### Discussion

Immobilization causes remarkable BBB damages here shown by Evans's Blue dye detection. In fact, clear differences are reported between the experimental group subjected to bioactive *R_{S}*-GRA injection and the one untreated with pharmacological therapy, in which more severe levels of extravasation have been detected, as a marker of BBB modified permeation. This alteration in physiological TJ expression causes the disruption of its proteic components. Among them, claudins represent the major barrier component, since they - together with occludin, ZO family members and other cytoplasmic proteins - maintain cell polarity via apical and basolateral domains in the plasma membrane. In particular, it was observed a considerable BBB breakdown evidenced by the claudin-1, claudin-3 and ZO-1 signals that appeared absent at the level of vascular endothelium of BBB. In the present work, iNOS as well as nitrotyrosine expression have been chosen as proof 1) of the activation of an oxidative stress mechanism during experimental stress, 2) to demonstrate that bioactive *R_{S}*-GRA can modulate the antioxidant pathway and 3) to confirm that BBB is protected by our treatment against reactive radicalic species. All hypotheses have been validated by our results, supporting the idea of the active role of the molecule counteracting the molecular basis of oxidative cascade. Moreover, the study was designed to verify whether an involvement of NF-kBp65 pathway after restraint stress could cause alterations in BBB functioning.

The activation of NF-kB can directly affect BBB alterations, increasing the paracellular permeability and altering key TJ proteins. Our data clearly correlate the oxidative stress and barrier dysfunction through the involvement of NF-kB pathway and confirm that bioactive *R_{S}*-GRA treatment has a key role in the process underlying these events. Moreover, this also suggests the anti-inflammatory power of bioactive *R_{S}*-GRA in mitigating inflammatory response by reducing the production of pro-inflammatory cytokine induced by NF-kB, such as TNF-α and IL-1β. It has been demonstrated that TNF-α and IL-1β interfere with BBB permeability. TNF-α seems to facilitate the adhesion, transmigration and permeability of leukocytes across the monolayer. Additionally, it upregulates the expression of ICAM-1 and VCAM-1 and induces apoptosis in cerebral endothelial cells. It has been suggested that TNF-α causes BBB breakdown, functioning through disruption of TJ assembly, leukocyte recruitment and direct damage to cerebral microvasculature. Also, several evidences from animal studies demonstrate that IL-1β also mediates the breakdown of the BBB and that TJ proteins (claudin-5 and ZO-1 and ZO-2) are degraded upon IL-1β challenge. A significant increase in TNF-α and IL-1β was clearly shown during restraint stress. On the contrary, no significant expression of TNF-α and IL-1β was observed in stressed mice treated with bioactive *R_{S}*-GRA. Furthermore, bioactive *R_{S}*-GRA seems also to modulate the expression of anti-inflammatory cytokines such as IL-10. In fact, it has been demonstrated a significant increase in the levels of IL-10 in stressed animals treated with bioactive *R_{S}*-GRA showing that it can play an important role in the regulation of anti-inflammatory cytokines. Moreover, notably, ITCs are able to block the degradation of Nrf2, identified as the main transcription factor that regulates cellular defense response through antioxidant response elements in normal tissues. Administration of bioactive *R_{S}*-GRA in stressed mice causes an upregulation of Nrf2 into the nucleus. In response, a protective action of Nrf2 on astrocytes occurs, causing a down-regulated GFAP expression according to other studies.

This balance avoids that GFAP expressing astrocytes may regulate the integrity of the barrier, damaging TJ components and interfering with the normal astrocyte interactions. It was observed that, after stress input, bioactive *R_{S}*-GRA treatment is able to induce a, possibly Nrf2-dependent, GFAP reduction. Also, to better understand the mechanism of action of bioactive *R_{S}*-GRA, it was evaluated whether following stress induction there are changes in proteins involved in the process of cell death. Thus, parallel to the known properties of glucosinolates *R_{S}*-GRA, also considerable variations here were found in stressed mice treated with bioactive *R_{S}*-GRA, analyzing the apoptotic pathway mediated by caspase-3, with a decrease in the expression levels of this protein. On the basis of these observations, together with reported restoration of TJ organization mediated by bioactive *R_{S}*-GRA treatment, an evident beneficial role of this phytochemical treatment can be acknowledged into preserving BBB organization due to its anti-oxidant and antiapoptotic effects. This is reflected in the improvement of the stress parameters, as it was shown by histological examination. Summarizing, our data suggest that bioactive *R_{S}*-GRA has pharmacological properties to protect BBB dysfunctions.

## Claims

1. Composition comprising *R_{S}*-Glucoraphanin and myrosinase as a neuroprotective agent, for use in preventing and treating neurodegenerative diseases; said composition further comprising a buffered solution at pH 6-8; wherein *R_{S}*-Glucoraphanin and myrosinase are reacted and contacted from 10 to 60 minutes before administration.

2. The composition for use of claim 1, wherein said neurodegenerative diseases are alterations in the permeability of the blood brain barrier tight junction, vascular dementia, brain ischemia/reperfusion injury, Parkinson disease, Alzheimer disease, multiple sclerosis, amyotrophic lateral sclerosis and Huntington disease.

3. The composition for use of claim 1 or 2, wherein said *R_{S}*-Glucoraphanin has a purity higher than 10%, preferably higher than 50% weight basis.

4. The composition for use of claim 1 or 2, wherein said *R_{S}*-Glucoraphanin has a purity higher than 90%, preferably higher than 95% weight basis.

5. The composition for use of any one of claims 1-4, wherein said *R_{S}*-Glucoraphanin is obtainable by extraction from Brassica oleracea L. var. *acephala sabellica,* said extraction comprising the following steps:
i) grinding the seeds of Brassica oleracea L. var. *acephala sabellica* to a fine powder and defatting with hexane, or grinding the sprouts of Brassica oleracea L. var. *acephala sabellica* to a fine powder,
ii) treating with boiling 70% ethanol,
iii) extracting glucosinolates by using an homogeniser and centrifuging the extract,
iv) isolating *R_{S}*-Glucoraphanin from the glucosinates' extract by one-step anion exchange chromatography, and optionally
v) purifying the isolated *R_{S}*-Glucoraphanin by gel-filtration.

6. The composition for use of any one of claims 1-5, wherein said *R_{S}*-Glucoraphanin is administered in an amount of 10-500 mg/day and said myrosinase in an amount of 0.4-120 U/day.

7. The composition for use of claim 6, wherein said *R_{S}*-Glucoraphanin and said myrosinase are contacted and reacted from 15 to 60 minutes, before administration.

8. The composition for use of claim 6 or 7, wherein said *R_{S}*-Glucoraphanin is administered in an amount of 50-250 mg/day.

9. The composition for use of any one of claims 1-8, wherein said composition is administered by oral, parenteral, inhalating, aerosol, spray, transdermal, intravenous, intramuscular, transdermal, subcutaneous, intraperitoneal, intranasal, intrarectal, sublingual or topical route, preferably by oral route.

10. The composition for use of claim 1, consisting of *R_{S}*-Glucoraphanin, myrosinase, and pharmacological acceptable carriers.

11. A kit for providing the composition for use of claim 1, comprising:
a) a container comprising *R_{S}*-Glucoraphanin, wherein *R_{S}*-Glucoraphanin in container a) is in solid powdered form or in a buffered solution at pH 6-8,
b) a container comprising myrosinase, wherein the container b) comprises a saline solution of myrosinase having pH 6-8, and
c) a leaflet comprising instructions for simultaneous, separate or sequential use of *R_{S}*-Glucoraphanin and myrosinase, in preventing and treating neurodegenerative diseases.

12. The kit of claim 11, wherein myrosinase is at a concentration from 5 to 50 U/ml.

13. The kit of claim 11, wherein *R_{S}*-Glucoraphanin in container a) is in a Phosphate-Buffered Saline (PBS) solution at pH 7.2.

## Patentansprüche

1. Zusammensetzung mit R_{S}-Glucoraphanin und Myrosinase als neuroprotektive Lösung zur Verwendung bei der Vorsorge und Behandlung neurodegenerativer Erkrankungen, wobei die Zusammensetzung des Weiteren eine gepufferte Lösung mit einem pH-Wert von 6 - 8 aufweist und R_{S}-Glucoraphanin und Myrosinase 10 bis 60 Minuten vor Verabreichung zusammengeführt werden und reagieren können.

2. Zusammensetzung zur Verwendung gemäß Anspruch 1, wobei die neurodegenerativen Erkrankungen Schwankungen in der Durchlässigkeit der Blut-Hirn-Schranken-Tight-Junction, vaskuläre Demenz, Hirn-Ischämie / Reperfusionsschaden, Parkinson-Erkrankung, Alzheimer, Multiple Sklerose, amyotrophe Lateralsklerose und Huntington-Erkrankung sein können.

3. Zusammensetzung zur Verwendung gemäß Anspruch 1 oder 2, wobei das Rs-Glucoraphanin eine Reinheit von mehr als 10%, vorzugsweise von mehr als 50% auf Gewichtsbasis aufweist.

4. Zusammensetzung zur Verwendung gemäß Anspruch 1 oder 2, wobei das Rs-Glucoraphanin eine Reinheit von mehr als 90%, vorzugsweise von mehr als 95% auf Gewichtsbasis aufweist.

5. Zusammensetzung zur Verwendung gemäß einem der Ansprüche 1 bis 4, wobei das Rs-Glucoraphanin durch Extraktion aus Brassica oleracea L. var. *acephala sabellica* gewonnen wird und die Extraktion folgende Schritte aufweist:
i) Mahlen der Samen von Brassica oleracea L. var. *acephala sabellica* zu feinem Pulver und Entfettung mittels Hexan oder Mahlen der Sprossen von Brassica oleracea L. var. *acephala sabellica* zu feinem Pulver,
ii) Behandlung mit kochendem 70%-igem Ethanol,
iii) Extraktion von Glucosinolaten unter Verwendung eines Homogenisators und Zentrifugieren des Extrakts;
iv) Isolation von R_{S}-Glucoraphanin aus dem Glucosinolat-Extrakt mittels Anionenaustauscher-Chromatographie und optional
v) Reinigung des isolierten R_{S}-Glucoraphanins mittels Gelfiltration.

6. Zusammensetzung zur Verwendung gemäß einem der Ansprüche 1 bis 5, wobei das Rs-Glucoraphanin in einer Menge von 10 - 500 mg/Tag und die Myrosinase in einer Menge von 0,4 - 120 U/Tag verabreicht wird.

7. Zusammensetzung zur Verwendung gemäß Anspruch 6, wobei R_{S}-Glucoraphanin und Myrosinase 15 bis 60 Minuten vor Verabreichung zusammengeführt werden und reagieren können.

8. Zusammensetzung zur Verwendung gemäß Anspruch 6 oder 7, wobei R_{S}-Glucoraphanin in einer Menge von 50 - 250 mg/Tag verabreicht wird.

9. Zusammensetzung zur Verwendung gemäß einem der Ansprüche 1 bis 8, wobei die Zusammensetzung oral, parenteral, durch Inhalation, als Aerosol, als Spray, transdermal, intravenös, intramuskulär, subkutan, intraperitoneal, intranasal, intrarektal, sublingual oder topisch verabreicht wird, vorzugsweise oral.

10. Zusammensetzung zur Verwendung gemäß Anspruch 1, umfassend R_{S}-Glucoraphanin, Myrosinase und pharmakologisch annehmbare Trägerstoffe.

11. Set zur Bereitstellung einer Zusammensetzung zur Verwendung gemäß Anspruch 1, aufweisend:
a) einen Behälter mit R_{S}-Glucoraphanin, wobei R_{S}-Glucoraphanin im Behälter a) in fester Pulverform oder in einer gepufferten Lösung mit einem pH-Wert von 6 - 8 vorliegt,
b) einen Behälter mit Myrosinase, wobei der Behälter b) eine Salzlösung von Myrosinase mit einem pH-Wert von 6 - 8 aufweist,
c) ein Informationsblatt mit Anleitungen zur simultanen, gesonderten oder sequenziellen Verwendung von R_{S}-Glucoraphanin und Myrosinase bei der Vorsorge und Behandlung neurodegenerativer Erkrankungen.

12. Set gemäß Anspruch 11, wobei Myrosinase in einer Konzentration von 5 bis 50 U/ml vorliegt.

13. Set gemäß Anspruch 11, wobei R_{S}-Glucoraphanin im Behälter a) in einer phosphatgepufferten Salzlösung (PBS) mit einem pH-Wert von 7,2 vorliegt.

## Revendications

1. Composition comprenant de la Rs-glucoraphanine et de la myrosinase en tant qu'agent neuroprotecteur, pour une utilisation dans la prévention et le traitement de maladies neurodégénératives ; ladite composition comprenant en outre une solution tamponnée à pH 6 à 8 ; dans laquelle la Rs-glucoraphanine et la myrosinase sont mises à réagir et en contact de 10 à 60 minutes avant l'administration.

2. Composition pour une utilisation selon la revendication 1, où lesdites maladies neurodégénératives sont des altérations de la perméabilité des jonctions serrées de la barrière hématoencéphalique, la démence vasculaire, la lésion d'ischémie/reperfusion cérébrale, la maladie de Parkinson, la maladie d'Alzheimer, la sclérose en plaques, la sclérose latérale amyotrophique et la maladie de Huntington.

3. Composition pour une utilisation selon la revendication 1 ou 2, dans laquelle ladite *Rs-*glucoraphanine présente une pureté supérieure à 10 %, de préférence supérieure à 50 % sur la base du poids.

4. Composition pour une utilisation selon la revendication 1 ou 2, dans laquelle ladite *R_{S}*-glucoraphanine présente une pureté supérieure à 90 %, de préférence supérieure à 95 % sur la base du poids.

5. Composition pour une utilisation selon l'une quelconque des revendications 1 à 4, dans laquelle ladite Rs-glucoraphanine peut être obtenue par extraction à partir de *Brassica oleracea* L. var. *acephala sabellica,* ladite extraction comprenant les étapes suivantes :
i) le broyage des graines de *Brassica oleracea* L. var. *acephala sabellica* en une poudre fine et le dégraissage avec de l'hexane, ou le broyage des germes de *Brassica oleracea* L. var. *acephala sabellica* en une poudre fine,
ii) le traitement avec de l'éthanol à 70 % en ébullition,
iii) l'extraction des glucosinolates en utilisant un homogénéiseur et la centrifugation de l'extrait,
iv) l'isolement de la Rs-glucoraphanine à partir de l'extrait des glucosinolates par une chromatographie avec échange d'anions en une étape, et éventuellement
v) la purification de la Rs-glucoraphanine isolée par filtration sur gel.

6. Composition pour une utilisation selon l'une quelconque des revendications 1 à 5, dans laquelle ladite Rs-glucoraphanine est administrée dans une quantité de 10 à 500 mg/jour et ladite myrosinase dans une quantité de 0,4 à 120 U/jour.

7. Composition pour une utilisation selon la revendication 6, dans laquelle ladite Rs-glucoraphanine et ladite myrosinase sont mises en contact et à réagir de 15 à 60 minutes, avant l'administration.

8. Composition pour une utilisation selon la revendication 6 ou 7, dans laquelle ladite *R_{S}*-glucoraphanine est administrée dans une quantité de 50 à 250 mg/jour.

9. Composition pour une utilisation selon l'une quelconque des revendications 1 à 8, où ladite composition est administrée par voie orale, parentérale, inhalation, aérosol, pulvérisation, transdermique, intraveineuse, intramusculaire, transdermique, sous-cutanée, intrapéritonéale, intranasale, intrarectale, sublinguale ou topique, de préférence par voie orale.

10. Composition pour une utilisation selon la revendication 1, constituée de Rs-glucoraphanine, de myrosinase, et de supports pharmacologiques acceptables.

11. Kit pour fournir la composition pour une utilisation selon la revendication 1, comprenant :
a) un récipient comprenant la Rs-glucoraphanine, dans lequel la Rs-glucoraphanine dans le récipient a) est sous forme de poudre solide ou dans une solution tamponnée à pH 6 à 8,
b) un récipient comprenant la myrosinase, dans lequel le récipient b) comprend une solution saline de myrosinase ayant un pH de 6 à 8, et
c) une notice comprenant des instructions pour une utilisation simultanée, séparée ou séquentielle de la Rs-glucoraphanine et de la myrosinase, dans la prévention et le traitement de maladies neurodégénératives.

12. Kit selon la revendication 11, dans lequel la myrosinase est à une concentration de 5 à 50 U/ml.

13. Kit selon la revendication 11, dans lequel la Rs-glucoraphanine dans le récipient a) est dans une solution tampon phosphate (PBS) à pH 7,2.
